(19) Europäisches Patentamt
European Patent Office
Office européen des brevets

(11) EP 1 307 583 B1

(12) **EUROPÄISCHE PATENTSCHRIFT**

(45) Veröffentlichungstag und Bekanntmachung des Hinweises auf die Patenterteilung:
**20.07.2005 Patentblatt 2005/29**

(21) Anmeldenummer: **01962825.4**

(22) Anmeldetag: **04.07.2001**

(51) Int Cl.7: **C12Q 1/00**, C12Q 1/26, C12Q 1/32, C12Q 1/42, G01N 33/543

(86) Internationale Anmeldenummer:
**PCT/EP2001/007626**

(87) Internationale Veröffentlichungsnummer:
**WO 2002/002796 (10.01.2002 Gazette 2002/02)**

(54) **ELEKTROCHEMISCHER EINWEGBIOSENSOR FÜR DIE QUANTITATIVE BESTIMMUNG VON ANALYTKONZENTRATIONEN IN FLÜSSIGKEITEN**

DISPOSABLE ELECTROCHEMICAL BIO-SENSOR FOR THE QUANTITATIVE DETERMINATION OF ANALYTE CONCENTRATIONS IN FLUIDS

BIOCAPTEUR ELECTROCHIMIQUE A USAGE UNIQUE PERMETTANT LA DETERMINATION QUANTITATIVE DE CONCENTRATIONS D'ANALYTES DANS DES LIQUIDES

(84) Benannte Vertragsstaaten:
**AT BE CH CY DE DK ES FI FR GB GR IE IT LI LU MC NL PT SE TR**

(30) Priorität: **05.07.2000 DE 10032042**

(43) Veröffentlichungstag der Anmeldung:
**07.05.2003 Patentblatt 2003/19**

(73) Patentinhaber: **Albatros Technologies GmbH & Co. KG**
**48161 Münster (DE)**

(72) Erfinder:
- **BARTETZKO, Norbert**
  **59071 Hamm (DE)**
- **BARTETZKO, Robert**
  **59071 Hamm (DE)**
- **KOCH, Jan, Hendrik**
  **48161 Münster (DE)**
- **MÜLLER, Ulrich**
  **44287 Dortmund (DE)**
- **SCHMIDLIN, Yvonne**
  **49082 Osnabrück (DE)**
- **SPECHT, Annemarie**
  **58456 Witten (DE)**

(74) Vertreter: **Leifert & Steffan**
**Patentanwälte,**
**Burgplatz 21-22**
**40213 Düsseldorf (DE)**

(56) Entgegenhaltungen:
**EP-A- 0 745 843**
**WO-A-97/15827**
**WO-A-99/19507**
**WO-A-99/58709**
**DE-A- 19 806 642**
**US-A- 5 609 749**
**US-A- 6 063 259**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 307 583 B1

Printed by Jouve, 75001 PARIS (FR)

## Beschreibung

Die vorliegende Erfindung betrifft einen Einwegbiosensor umfassend ein Trägermaterial, elektrische Leiterbahnen, ein Elektrodensystem umfassend eine Gegenelektrode und eine aus einer Reaktionsschicht gebildete Arbeitselektrode, eine dielektrische Isolatorschicht, welche das Trägermaterial sowie die elektrischen Leiterbahnen bedeckt und Aussparungen für die Kontaktierung einer Potentiostateinheit und für das Elektrodensystem aufweist, sowie eine analyterkennende Biokomponente. Die Erfindung betrifft außerdem Verfahren zur Bestimmung von Analyten in einer flüssigen Probe unter Verwendung der erfindungsgemäßen Einwegbiosensoren, die Verwendung leicht sublimierender Verbindungen als Elektronentransfermediatoren eines elektrochemischen Sensors zur Überführung von Elektronen von einem Enzym zu einem elektronenleitenden Material, sowie die Verwendung der erfindungsgemäßen Einwegbiosensoren zur Bestimmung von Analytkonzentrationen in Körper- oder Probenflüssigkeiten.

Elektrochemische Detektionssysteme auf der Basis mediatormodifizierter Biosensoren zur spezifischen quantitativen Bestimmung eines Analyten in einer flüssigen Probe sind in der Literatur und in vielen Patenten bereits ausführlich beschrieben worden (zum Beispiel in der EP 0 552 223, US 5,288,636, US 4,711,245). Dabei handelt es sich zumeist um Biosensoren für Analyte, die durch Enzyme der Klasse der Oxidoreduktasen spezifisch umgesetzt werden können (Laktat, Cholesterol, Ethanol, Wasserstoffperoxid, Hamsäure usw.). Teilweise sind mediatormodifizierte Biosensoren bereits als kommerzielle Produkte erhältlich. Das bekannteste Beispiel eines solchen Biosensors ist der Glukosebiosensor, der in der medizinischen Diagnostik von Diabetikern genutzt wird, um Glukosekonzentrationen im Vollblut zu bestimmen. Das Detektionsprinzp eines mediatormodifizierten Biosensors ist zum Beispiel in der EP 0 552 223 für einen Glukosebiosensor beschrieben.

Das in einem Glukosebiosensor verwendete Enzym ist die Glukoseoxidase (GOD). Die GOD besitzt eine prosthetische Gruppe in Form eines Flavinadenindinukleotid-Moleküls (FAD) und katalysiert die Reaktion der Glukose zu Glukonolakton gemäß Gleichung (I):

$$\text{(I)} \qquad \text{Glukose + GOD-FAD} \Leftrightarrow \text{Glukonolakton + GOD-FADH}_2$$

Grundsätzlich ist es nun möglich, in einer weiteren Redox-Reaktion das reduzierte Enzym (GOD-$FADH_2$) mit Sauerstoff ($O_2$) zu GOD-FAD unter Bildung von Wasserstoffperoxid ($H_2O_2$) gemäß Gleichung (IIa) zu oxidieren und anschließend durch die Bestimmung der Wasserstoffperoxid-Konzentration mittels einer Platinelektrode die Glukosekonzentration in der Probe direkt zu ermitteln.

$$\text{(IIa)} \qquad \text{GOD-FADH}_2 + O_2 \Leftrightarrow \text{GOD-FAD} + H_2O_2$$

In den meisten Ausführungen des Glukosebiosensors wird jedoch ein elektronenübertragender Mediator verwendet, um die Glukosemenge in der Probe zu quantifizieren. Dieser Mediator oxidiert in einem zweiten Reaktionsschritt das Enzym, so dass dieses für die Reaktion (I) wieder zur Verfügung steht:

$$\text{(IIb)} \qquad \text{GOD-FADH}_2 + \text{MED}_{ox} \Leftrightarrow \text{GOD-FAD} + \text{MED}_{red}$$

Die Glukosekonzentration in der Probe läßt sich nun bestimmen, indem man in einer amperometrischen Messung den reduzierten Mediator ($MED_{red}$) an der Arbeitselektrode des Biosensors wieder oxidiert und den dabei fließenden Strom quantifiziert:

$$\text{(III)} \qquad \text{MED}_{red} \Leftrightarrow \text{MED}_{ox} + e^-$$

Um diese Reaktion ablaufen zu lassen, benötigt man eine Potentiostateinheit, die ein geeignetes Meßpotential vorgibt und den fließenden Strom mißt. Der Strom ist dabei proportional zur Konzentration der Glukose in der Probe.

Das Detektionsprinzip eines mediatormodifizierten Biosensors hat gegenüber der direkten Quantifizierung des Wasserstoffperoxids an einer Platinelektrode den Vorteil, dass bei einem niedrigen Arbeitspotential (0-350 mV vs. Ag/AgCl) gearbeitet werden kann. Dies dient der Verminderung, im Idealfall sogar der Vermeidung von störenden Interferenzen, die durch die Oxidation von Begleitsubstanzen entstehen können (Störsubstanzen im Blut sind beispielsweise Ascorbinsäure, Harnsäure, oder Paracetamol).

Eine weitere Möglichkeit, modifizierte Biosensoren zu nutzen, besteht darin, Analyte, die sich spezifisch in einem enzymmarkierten Assay einbinden lassen, zu quantifizieren. Dazu wird der Analyt spezifisch an ein auf der

Oberfläche des Sensors immobilisiertes Fängermolekül (analyterkennende Biokomponente) gebunden. An diesen Komplex aus Fängermotekül und Analyt wird wiederum ein mit einem Enzym gelabeltes Molekül gekoppelt. Die Konzentration des eingebundenen Analyten läßt sich bestimmen, indem man die Enzymmenge, die über den Assay an der Oberfläche des Biosensors immobilisiert ist, quantifiziert. Dies erfolgt durch Zugabe einer definierten Konzentration eines Substrates, welches durch das Enzym umgesetzt wird. Der Mediator des Biosensors regeneriert das an den Assay gebundene Enzym entsprechend Gleichung (IIb). Die Detektionsreaktion ist wiederum die Oxidation bzw. Reduktion des Mediators an der Biosensoroberfläche (siehe Gleichung III). Der dabei zu messende Strom ist proportional zur gelabelten Enzymmenge und damit auch proportional zur im Assay gebundenen Analytmenge. Dieses Sensorprinzip wird beispielsweise in der Immunosensorik oder der DNA-Analytik angewendet.

Verbindungen, die als Mediatoren in den Biosensoren eingesetzt werden, sollten sich dadurch auszeichnen, dass sie ein niedriges Redoxpotential aufweisen, dass sie hohe Umsetzungsraten mit dem Enzym erreichen, dass sie im reduzierten Zustand nicht leicht löslich sind und dass sie stabil und inert gegenüber anderen Begleitsubstanzen sind. In der Literatur und in zahlreichen Patenten sind viele Verbindungen publiziert, die sich als Mediatoren in Biosensoren einsetzen lassen. Eine große Gruppe von Mediatoren stellen metallorganische Verbindungen dar. Beispiele dafür sind Ferrocen-Derivate (Britisches Patent 8132034, US 4,711,245), Übergangsmetallkomplexe (US 5,710,011), z. B. Kaliumhexacyanoferrat (US 5,645,709), Nickel- oder Kobaltbipyridyl sowie Osmium enthaltende organische Verbindungen (US 5,846,702). Diese metallorganischen Mediatoren weisen jedoch den Nachteil auf, dass sie eine vergleichsweise geringe Umsetzungsrate aufweisen und dementsprechend in hohen Konzentrationen eingesetzt werden müssen. Dies ist ungünstig vor allem für die Verwendung dieser Mediatoren in Einwegbiosensoren in Form von Teststreifen. Ein weiterer Nachteil der metallorganischen Mediatoren ist ihre gute Löslichkeit in Wasser. Diese hat zur Folge, dass die Mediatoren bei einem Kontakt mit der Probeflüssigkeit relativ leicht aus dem Elektrodenraum herausdiffundieren können, so dass sich zum einen die Sensitivität des Biosensors aufgrund einer Abnahme der Mediatorkonzentration im Elektrodenraum verschlechtert und es zum anderen durch den Kontakt der Mediatoren mit Begleitsubstanzen, die vor allem bei der Analyse von Blutproben vorhanden sind, zu störenden Nebenreaktionen kommen kann. Vorteilhafter sind daher Verbindungen, die eine geringere Löslichkeit aufweisen und auch in geringerem Umfang mit Begleitsubstanzen wechselwirken.

Diese gewünschten, vorteilhaften Eigenschaften werden in der Regel von Molekülen erfüllt, die chinoide Strukturen ausbilden (Hydrochinon/Benzochinon, p-Aminophenol/p-Iminochinon) und die daher ebenfalls als Mediatoren in Biosensoren eingesetzt werden. Häufig verwendete Verbindungen sind auch das Tetrathiafulvalen (TTF), Tetracyanoquinodimethan (TCNQ) oder N-Methyl-Phenazinium (NMP) (US 5,876,952). Viele dieser Verbindungen zeichnen sich vor allem aufgrund ihrer photosensorischen Eigenschaften als gute Elektronendonatoren bzw. -akzeptoren aus und werden daher häufig als Mediatoren in Biosensoren eingesetzt. Zu nennen sind in diesem Zusammenhang beispielsweise N,N,N',N'-tetrakis-(2'hydroxyethyl)-p-phenylendiamin (THEPD) und N,N,N',N'-tetrakiscarboxymethyl-p-phenylendiamin (TCPD) (US 5,609,749) sowie 1,4-Diamino-2,3,5,6-tetramethylbenzen, 2,5-Diethyl-1,4-bis(dimethylaminobenzen), N-(4-Morpholinophenylhexahydroazepin), Meldolablau u. a. (WO 9207263). Diese Moleküle sind im wesentlichen Derivate des Phenylendiamins (PPD), das, wie auch das N,N,N',N'-Tetraphenylendiamin (TMPD), ebenfalls gute Donator/Akzeptoreigenschaften aufweist (J. Anal. Chem. USSR 45 (1990)). Im Vergleich zu den metallorganischen Mediatoren zeichnen sich die Derivate des PPDs zwar durch eine erhöhte Umsetzungsrate sowie eine geringere Löslichkeit aus, der Nachteil dieser leicht sublimierenden Verbindungen besteht jedoch darin, dass sie einen verhältnismäßig hohen Dampfdruck besitzen. Dies führt dazu, dass der Mediator, nachdem er auf das Substrat des Biosensors aufgetragen wurde, sich im Laufe der Zeit verflüchtigt und so die Sensitivität des Biosensors bei einer längeren Lagerung abnimmt. Problematisch ist des Weiteren die Tatsache, dass die Derivate des PPDs aufgrund einer wenn auch im Vergleich zu den metallorganischen Mediatoren geringen Löslichkeit in Wasser bei einem Kontakt mit der wässrigen Probenflüssigkeit aus dem Elektrodenraum herausdiffundieren und folglich durch die Abnahme der Mediatorkonzentration eine geringere Sensitivität des Biosensors beobachtet wird.

Die der vorliegenden Anmeldung zugrunde liegende Aufgabe bestand demnach in der Entwicklung eines mediatormodifizierten Biosensors, der trotz der Verwendung leicht sublimierender Verbindungen als Mediatoren die oben genannten Nachteile eines Sensitivitätsverlustes aufgrund einer längeren Lagerung oder als Folge des Kontaktes mit der Probenflüssigkeit nicht aufweist.

Die Aufgabe wurde erfindungsgemäß dadurch gelöst, dass in einem Biosensor der eingangs genannten Art das Elektrodensystem mit einer polymeren Schutzschicht versehen wurde. Durch diese Schutzschicht läßt sich nicht nur die Sensitivität des Biosensors steigern, indem die Schutzschicht den Mediator, der sich aus der Elektrode löst, in Elektrodennähe hält, sondern vor allem auch eine deutliche Verbesserung der Langzeitstabilität des Biosensors trotz der Verwendung leicht sublimierender Mediatoren erzielen. Dies ist vor allem darauf zurückzuführen, dass die polymere Schutzschicht der Verdampfung des Mediators entgegenwirkt. Auch läßt sich durch die Wirkung dieser polymeren Schutzschicht als Diffusionsbarriere der lineare Meßbereich des Biosensors verlängern.

Viele in Publikationen und Patenten vorgestellte Biosensoren sind aus den unterschiedlichsten Gründen mit polymeren Schichten versehen worden. So werden beispielsweise polymere Membranen verwendet, um die Enzyme

auf der Sensoroberfläche zu immobilisieren (EP 0 851 244, EP 0 894 869, EP 0 856 586, EP 0 909 952). In anderen Anwendungen wird mit Hilfe eines Polymers eine sogenannte Reaktionsschicht über der Arbeitselektrode aufgebaut, die sowohl den Mediator als auch das Enzym enthält (Patente der Matsushita Electric Ind. Co. LTD, JP, z.B.: US 5,192,415, EP 0 901 018, WO 9835225). Polymere Schichten können darüber hinaus dazu dienen, Interferenzen durch Störsubstanzen zu vermeiden oder die Sensoroberfläche vor Kontaminationen zu schützen (letzteres ist besonders bei Mehrwegbiosensoren und der Entwicklung von *in vivo* Sensoren von großer Bedeutung). Nafionschichten werden häufig verwendet, um Interferenzen durch anionische Störsubstanzen (z. B. Ascorbat) zu verringern (US 5,312,590). Eine Schicht aus Polyacrylamid kann dazu dienen, den Einfluß des Hämatocrit einer Blutprobe auf das Sensorsignal zu reduzieren (EP 0 769 558). Letztendlich lassen sich polymere Schichten auch dazu nutzen, eine Passivierungsschicht zu erzeugen, die unspezifische Bindungen an der Sensorelektrode verhindert (DE 198 06 642). Die Verwendung einer polymeren Schutzschicht zur Verhinderung des Verdampfens eines leicht sublimierenden Mediators und somit auch zur Verbesserung der Langzeitstabilität von Biosensoren ist hingegen bisher in der Literatur noch nicht beschrieben worden.

Die Gegenstand der vorliegenden Erfindung betrifft einen Einwegbiosensor umfassend ein Trägermaterial, elektrische Leiterbahnen, ein Elektrodensystem umfassend eine Gegenelektrode und eine aus einer Reaktionsschicht gebildete Arbeitselektrode, eine dielektrische Isolatorschicht, welche das Trägermaterial sowie die elektrischen Leiterbahnen bedeckt und Aussparungen für die Kontaktierung einer Potentiostateinheit und für das Elektrodensystem aufweist, sowie eine analyterkennende Biokomponente. Der erfindungsgemäß Einwegbiosensor ist dadurch gekennzeichnet, dass die Reaktionschicht einen leicht sublimierenden Mediator enthält und dass das Elektrodensystem von einer polymeren Schutzschicht bedeckt ist. Der erfindungsgemäße Einwegbiosensor wird in der Form eines Chips hergestellt und in Kombination mit einer Potentiostateinheit angewendet. Er dient zur schnellen Vor-Ort-Quantifizierung von Analytkonzentrationen in Flüssigkeiten. Die nachweisbaren Analyte sind Substanzen, die sich spezifisch durch Oxidoreduktasen umsetzen oder sich in einem enzymmarkierten Immuno- oder Rezeptorassay einbinden lassen. Die Quantifizierung erfolgt amperometrisch oder zyklovoltametrisch.

Der Aufbau einer der bevorzugten Ausführungsformen des erfindungsgemäßen Biosensors ist als Beispiel in der Abbildung 1 dargestellt. Der Biosensor umfaßt ein Trägermaterial (1), auf dem nacheinander elektrische Leiterbahnen (2), eine Isolatorschicht (3), ein Elektrodensystem umfassend eine Gegenelektrode (6) und eine Arbeitselektrode (5), sowie eine das Elektrodensystem bedeckende polymere Schutzschicht (8) vorzugsweise durch Masken- oder Siebdruck aufgebracht worden sind, sowie eine analyterkennende Biokomponente. Die Arbeitselektrode (5) wird aus einer einen leicht sublimierenden Mediator sowie ein elektronenleitendes Material enthaltenden Reaktionsschicht gebildet. Die Leiterbahnen dienen dazu, die jeweiligen Elektroden mit den Anschlußpunkten (4), über die der Biosensor mit der Potentiostateinheit kontaktiert wird, zu verbinden. Die Isolatorschicht (3) bedeckt die Leiterbahnen und weist Aussparungen für die Anschlußpunkte (4) und sowie für das Elektrodensystem auf und dient der Verhinderung von parasitären Strömen. In der in dieser Abbildung 1 dargestellten bevorzugten Ausführungsform des erfindungsgemäßen Einwegbiosensors umfaßt das Elektrodensystem neben der Gegenelektrode (6) und der Arbeitselektrode (5) noch eine Referenzelektrode (7). Durch die Verwendung dieser Referenzelektrode kann eine deutliche Verbesserung der Meßgenauigkeit erzielt werden.

Die Leiterbahnen (2) bestehen aus einer als wesentliche Bestandteile Kohlenstoff-, Silber-, Platin- oder Goldpartikel enthaltenden Polymerpaste. Die Leiterbahnen (2) werden vorzugsweise durch Sieb- oder Maskendruck auf das Trägermaterial (1) aufgebracht und anschließend ausgehärtet.

Das zur Herstellung der Einwegbiosensoren verwendete Trägermaterial (1) besteht bevorzugt aus Polycarbonat, Polyvinylchlorid (PVC), Polyethylenterephtalat (PET), Polypropylen, Polyester oder Polystyrol.

Zur Herstellung der Gegenelektrode (6) und der Referenzelektrode (7) wird bevorzugt eine Silberpartikel- und Silberchlorid-haltige Leitpaste, der ein leicht lösliches Chloridsalz (z. B. Natriumchlorid oder Kaliumchlorid) zugesetzt worden ist, verwendet. Die Gegenelektrode (6) und die Referenzelektrode (7) werden vorzugsweise durch Sieb- oder Maskendruck auf das Trägermaterial (1) aufgebracht und anschließend ausgehärtet.

Die Reaktionschicht, aus der die Arbeitselektrode (5) gebildet worden ist, enthält neben dem leicht sublimierenden Elektronentransfermediator ein elektronenleitendes Material, welches aus einer als wesentliche Bestandteile Kohlenstoff-, Platin-, Palladium-, Rhodium- oder Goldpartikel enthaltenden, aushärtbaren Polymerpaste besteht. Die Reaktionsschicht wird vorzugsweise durch Sieb- oder Maskendruck auf das Trägermaterial (1) aufgebracht und anschließend ausgehärtet.

Bei den in der vorliegenden Erfindung verwendeten leicht sublimierenden Mediatoren handelt es sich um Verbindungen, die einen Dampfdruck von mindestens $1 \cdot 10^{-5}$ mmHg bei 25°C aufweisen. Die Mediatoren besitzen vorzugsweise die in der Abbildung 2 dargestellte Struktur, in der die Reste $R^1$, $R^2$, $R^3$ und $R^4$ gleich oder verschieden sein können und ein Wasserstoffatom, einen $C_1$-$C_{10}$-Alkylrest, bevorzugt einen $C_1$-$C_5$-Alkylrest, oder einen Arylrest darstellen. Bevorzugte Mediatoren sind p-Aminodiphenylamin, p-Phenylendiamin oder N,N,N',N'-Tetramethyl-p-phenylendiamin.

Eine den Mediator stabilisierende hydrophile Schutzschicht (8) in Form eines polymeren Hydrogels, die der

Verdampfung des Elektronentransfermediators entgegenwirkt und gleichzeitig die Sensitivität des Biosensors erhöht und als Diffusionsbarriere fungiert, bedeckt das vollständige Elektrodensystem. Die vorzugsweise sieb- oder maskengedruckte polymere Schutzschicht (8) besteht im wesentlichen aus einem Hydrogel aus Polyvinylpyrrolidon (PVP), Polyethylenoxid (PEO), Stärke oder Gelatine.

Als analyterkennende Biokomponente kann ein Enzym, ein DNA-Molekül, ein Fänger-Antikörper oder ein Rezeptor-Molekül fungieren. Als Enzym wird bevorzugt eine Oxidoreduktase, eine Dehydrogenase, eine β-Galaktosidase oder eine alkalische Phosphatase verwendet. Besonders bevorzugte Enzyme sind Glukoseoxidase (GOD), Laktatoxidase (LOD), Uricase, Ascorbatoxidase, Ethanoloxidase, Cholesteroloxidase oder Peroxidase (POD).

In der in der Abbildung 1 dargestellten bevorzugten Ausführungsform des erfindungsgemäßen Biosensors ist des Weiteren über dem Elektrodensystem des Biosensors ein Kunststoffgewebe (9) mit einem einseitig klebenden Tape (10) befestigt. Das einseitig klebende Tape (10) weist eine Aussparung auf, in welche die flüssige Probe gebracht werden kann, so dass die Probe über das Kunststoffgewebe (9) die Elektroden, welche durch die Potentiostateinheit mit einer Meßspannung versorgt werden, kontaktiert. Dieses Gewebe dient jedoch nicht nur der schnellen und gleichmäßigen Verteilung der Probe über den Elektroden, sondern auch zum dem Schutz der Biosensoroberfläche vor Zerstörung oder Kontamination, die beispielsweise auftreten können, wenn als Probe Kapillarblut mit dem Finger aufgetragen wird. Das Kunststoffgewebe (9) besteht bevorzugt aus einem Polyethylen-, Polypropylen- oder Polyamidnetz, während das Tape (10) aus einer einseitig klebenden Polyester-, PVC- oder Papierklebefolie besteht.

Je nach Lokalisation der analyterkennenden Biokomponente und nach Struktur der Reaktionschicht können grundsätzlich fünf verschiedene Ausführungsformen des erfindungsgemäßen Biosensors unterschieden werden.

1. In einer ersten Ausführungsform des erfindungsgemäßen Biosensors besteht die Reaktionsschicht aus einer einzigen Schicht enthaltend die elektronenleitende Polymerpaste, den leicht sublimierenden Mediator sowie die analyterkennende Biokomponente.

2. In einer zweiten Ausführungsform des erfindungsgemäßen Biosensors besteht die Reaktionsschicht aus einer einzigen Schicht enthaltend die elektronenleitende Polymerpaste und den leicht sublimierenden Mediator. In dieser Ausführungsform ist die analyterkennende Biokomponente jedoch in der polymeren Schutzschicht (8) lokalisiert.

3. In einer dritten Ausführungsform des erfindungsgemäßen Biosensors besteht die Reaktionsschicht aus zwei Schichten, wobei die erste Schicht aus der elektronenleitenden Polymerpaste sowie dem leicht sublimierenden Mediator gebildet wird und von einer zweiten, hydrophilen polymeren Deckschicht bedeckt ist, die als Träger der analyterkennenden Biokomponente fungiert.

4. In einer vierten Ausführungsform des erfindungsgemäßen Biosensors besteht die Reaktionsschicht ebenfalls aus zwei Schichten, wobei in diesem Fall die erste Schicht aus der elektronenleitenden Polymerpaste gebildet wird und von einer zweiten, hydrophilen polymeren Deckschicht bedeckt ist, die als Träger der analyterkennenden Biokomponente und des leicht sublimierenden Mediators fungiert.

5. In einer fünften Ausführungsform des erfindungsgemäßen Biosensors besteht die Reaktionsschicht ebenfalls aus zwei Schichten, wobei die erste Schicht aus der elektronenleitenden Polymerpaste gebildet wird und von einer zweiten, hydrophilen polymeren Deckschicht bedeckt ist, die als Träger des leicht sublimierenden Mediators fungiert. Die analyterkennende Biokomponente ist in dieser Ausführungsform in der polymeren Schutzschicht (8) lokalisiert.

Die analyterkennende Biokomponente kann sowohl direkt oder aber über eine Avidin/Biotin-Wechselwirkung (siehe Abbildungen 18 und 20) in der Reaktionschicht oder aber in der polymeren Schutzschicht (8) immobilisiert sein.

Die gegebenenfalls in der Reaktionschicht enthaltende polymere Deckschicht besteht im wesentlichen aus einem Hydrogel aus Polyvinylpyrrolidon (PVP), Polyethylenoxid (PEO), Stärke oder Gelatine, welches bevorzugt durch Sieb- oder Maskendruck aufgedruckt worden ist.

Die vorliegende Erfindung betrifft auch ein Verfahren zur Bestimmung von Analytkonzentrationen in wässrigen Lösungen unter Verwendung der erfindungsgemäßen Einwegbiosensoren.

Bei der Verwendung derjenigen Ausführungsform des Biosensors, bei der die analyterkennende Biokomponente ein Enzym, z. B. Glukoseoxidase, Laktatoxidase, Alkoholoxidase, Peroxidase oder Uricase ist, wird eine flüssige Probe zunächst auf das Elektrodensystem des Biosensors gebracht. Der zu bestimmende Analyt, z. B. Glukose, wird dann durch das jeweilige Enzym, wie dies für den Fall des Glukose-Nachweises in Gleichung (I) beschrieben ist, reduziert. Das reduzierte Enzym reduziert dann in einem weiteren Reaktionsschritt entsprechend der Gleichung (IIb) den Mediator. Über eine Potentiostateinheit, die ein geeignetes Meßpotential vorgibt, wird dann der Mediator oxidiert und der dabei fließende Strom, der proportional zur Analytkonzentration ist, bestimmt.

Wenn der erfindungsgemäße Biosensor als enzymmarkierter Immunoassay, als DNA-Assay oder als Ligand-Assay eingesetzt wird, so wird auch in diesem Fall zunächst eine flüssige Probe auf das Elektrodensystem des Biosensors gebracht. Das in der Probenflüssigkeit enthaltende Antigen, das sequenzspezifische DNA-Molekül oder der Ligand binden dann an die in der Reaktionschicht oder der polymeren Schutzschicht (8) immobilisierte analyterkennende Biokomponente (Antikörper, DNA-Sonde oder Rezeptor). Anschließend wird die Sensoroberfläche gewaschen und danach mit einer Lösung bedeckt, die ein analytspezifisches Enzym-Konjugat enthält, welches die sequenzspezifische DNA, das Antigen oder den Liganden erkennt. Dieses Enzym-Konjugat bindet an den Analyten (Antikörper, DNA-Sonde oder Rezeptor) und die Menge des in der Reaktionschicht oder der polymeren Schutzschicht (8) gebundenem Enzym-Konjugates ist proportional zur Analytmenge in der zu Beginn aufgetragenen Probenflüssigkeit. Nach erneutem gründlichen Waschen der Oberfläche der Arbeitselektrode wird diese mit einer weiteren Lösung enthaltend ein Substrat des Enzym-Konjugates beschichtet. Das Substrat wird vom Enzym umgesetzt und das reduzierte oder oxidierte Enzym reagiert dann entsprechend Gleichung IIb) mit dem Mediator. Über eine Potentiostateinheit, die ein geeignetes Meßpotential vorgibt, wird dann der dabei fließende Strom bestimmt.

Die vorliegende Erfindung betrifft auch die Verwendung leicht sublimierender Verbindungen mit einem Dampfdruck von mindestens $1 \cdot 10^{-5}$ mmHg bei 25°C als Elektronentransfermediatoren zur Überführung von Elektronen von einem Enzym zu einem elektronenleitenden Material, wobei sich diese Mediatoren in einer Reaktionsschicht befinden, welche durch eine polymere Schutzschicht (8) bedeckt ist. Die polymere Schutzschicht (8) wirkt der Sublimation des Mediators entgegen, so dass sich die Langzeitstabilität des Sensors erheblich verlängern läßt. Bei den zur Überführung von Elektronen von einem Enzym zu einem elektronenleitenden Material bevorzugt verwendeten Mediatoren handelt es sich um leicht sublimierende Verbindungen mit der Struktur (I),

(I)

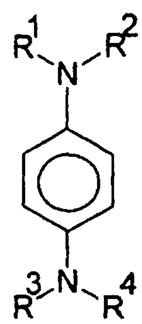

in der die Reste $R^1$, $R^2$, $R^3$ und $R^4$ gleich oder verschieden sein können und ein Wasserstoffatom, einen $C_1$-$C_{10}$-Alkylrest, bevorzugt einen $C_1$-$C_5$-Alkylrest, oder einen Arylrest darstellen. Bevorzugte zur Überführung von Elektronen von einem Enzym zu einem elektronenleitenden Material verwendete Mediatoren sind p-Aminodiphenylamin, p-Phenylendiamin oder N,N,N',N'-Tetramethyl-p-phenylendiamin.

Die vorliegende Erfindung betrifft des weiteren die Verwendung des erfindungsgemäßen Einwegbiosensors zur Bestimmung von Analytkonzentrationen in Körper- oder Probenflüssigkeiten. Bei den mit dem erfindungsgemäßen Biosensor nachweisbaren Analyten handelt es sich bevorzugt um Glukose, Laktat, Harnsäure, Ascorbat, Ethanol, Cholesterol oder Wasserstoffperoxid. Auch sequenzspezifische DNA-Fragmente, spezifische Antigene oder auch Liganden können mit besonderen Ausführungsformen des erfindungsgemäßen Biosensors nachgewiesen werden. Bei den Flüssigkeiten, die mit dem vorliegenden Biosensor analysiert werden können, handelt es sich um wäßrige synthetische Lösungen, Nährlösungen, oder aber auch um Blut, Blutserum, Blutplasma oder um Urin.

BEISPIELE

Allgemeine Hinweise zur Charakterisierung der Sensoren

Das Redox-Verhalten des im Einwegbiosensor eingesetzten Mediators läßt sich anhand von zyklischen Voltammogrammen in einer gepufferten Lösung (PBS, pH 7,4, 154 mM $Cl^-$) darstellen. Darin auftretende Peaks verdeutlichen die elektrochemische Oxidierbarkeit bzw. Reduzierbarkeit des Mediators. Die Peakpotentiale bestimmen das Arbeitspotential des Biosensors. Die Peakströme sind ein Maß für die oxidierte bzw. reduzierte Stoffmenge. Sinkende Peakströme nach längeren Lagerzeiten der Biosensoren deuten demnach auf den Verlust oder den Zerfall des Mediators hin.

Zur Überprüfung der Stabilität eines Mediators werden zyklische Voltammogramme in gepufferten wässrigen Lösungen in regelmäßigen Zeitabständen mit jeweils unbenutzten Biosensoren durchgeführt und die erhaltenen Peakströme verglichen.

Sowohl zyklische Voltammogramme als auch amperornetrische Messungen in analythaltigem Puffer zeigen die Fähigkeit des jeweiligen Mediators, das Enzym zu regenerieren und die Elektronen auf die Elektrode zu übertragen. Dies äußert sich in beiden Experimentformen in einer Erhöhung der Ströme gegenüber Messungen, die in analytfreiem Puffer durchgeführt werden. Mit Hilfe amperometrischer Messungen bei verschiedenen Analytkonzentrationen läßt sich die Sensitivität der Biosensoren ermitteln. Diese ist definiert als der Anstieg des Stromes mit größer werdender Konzentration. Sowohl die mit zunehmendem Sensoralter auftretende Änderung der Sensitivität als auch die Änderung der Peakströme im zyklischen Voltammogramm dienen der Charakterisierung der Langzeitstabilität.

A) <u>Biosensoren zur Bestimmung von Glukosekonzentrationen in Probenflüssigkeiten</u>

Mit dem zuvor beschriebenen Sensoraufbau lassen sich unterschiedlich gestaltete Glukosebiosensoren herstellen. Die verschiedenen Modifikationen dieser Glukosebiosensoren unterscheiden sich dabei unter anderem in der Wahl der Verbindung, die als Mediator eingesetzt wird, oder aber im Material der polymeren Schutzschicht. Als Enzym wird in den aufgeführten Beispielen 1-3 Glukoseoxidase (von *Aspergillus niger*) verwendet. Die schützende und sensitivitätssteigemde Wirkung der polymeren Schutzschicht wird verdeutlicht, indem Meßergebnisse von Biosensoren mit und ohne Schutzschicht einander gegenüber gestellt werden.

<u>Beispiel 1</u>

| | |
|---|---|
| Elektrodenmaterial: | Kohlenstoff-Paste |
| Mediator: | p-Aminodiphenylamin (ADPA) |
| Enzym: | Glukoseoxidase (*Aspergillus niger*) |
| Schutzschicht: | Polyvinylpyrrolidon (PVP) |
| Ausführungsform | Die Reaktionsschicht besteht aus einer einzelnen Schicht enthaltend ein elektronenleitendes Material, den Mediator und die analyterkennende Biokomponente (Glukoseoxidase) |

Der Peakstrom (Oxidationsstrom) eines ADPA-modifizierten Biosensors ohne Schutzschicht beträgt bei 200 mV (vs. Ag/AgCl) zu Beginn der Meßreihe 216 nA (Abbildung 3). Durch die signalerhöhende Wirkung einer PVP-Schutzschicht läßt sich der Peakstrom auf 2000 nA verstärken (Abbildung 4). Während die ADPA-Biosensoren ohne Schutzschicht innerhalb von 54 Tagen 55% ihrer oxidierbaren ADPA-Menge verlieren, bleibt diese Menge bei Biosensoren mit PVP-Schutzschicht in diesem Zeitraum konstant.

Die zyklische Voltammogramme in gepufferter Glukoselösung ($c_{Glukose}$=15 mM) zeigen, dass ADPA als Mediator geeignet ist. Der Strom im Potentialbereich der Oxidation von ADPA steigt durch die Anwesenheit von Glukose bei Biosensoren ohne Schutzschicht auf 550 nA, bei Biosensoren mit Schutzschicht auf annähernd 6000 nA. Während die ADPA-Biosensoren ohne Schutzschicht innerhalb von 54 Tagen deutliche Signalverluste aufweisen (Abbildung 5), bleibt der Signalwert der PVP-bedeckten Biosensoren in diesem Zeitraum konstant (Abbildung 6). Eine deutliche Verbesserung bewirkt die Verwendung der PVP-Schutzschicht in den ADPA-Biosensoren auch hinsichtlich der Sensitivität der Sensoren. Anfänglich beträgt die Sensitivität der ungeschützten ADPA-Biosensoren 204 nA/mM (Abbildung 7). Bei geschützten Biosensoren liegt die Sensitivität anfänglich bei 325 nA/mM (Abbildung 8). Während die PVP-Schutzschicht dafür sorgt, dass diese Sensitivität von 325 nA/mM auch nach 54 Tagen erhalten bleibt, sinkt sie bei den ungeschützten Biosensoren innerhalb von 54 Tagen bereits auf 136 nA/mM ab. Auffällig ist weiterhin die Verlängerung des linearen Meßbereichs des ADPA-Biosensors durch die PVP-Schutzschicht. Ohne diese Schutzschicht beobachtet man bei 4 mM Glukose bereits eintretende Sättigung des Sensorsignals, wohingegen bei geschützten Biosensoren diese Konzentration noch im linearen Bereich des Biosensors erfaßt wird.

<u>Beispiel 2</u>

| | |
|---|---|
| Elektrodenmaterial: | Kohlenstoff-Paste |
| Mediator: | N,N,N',N'-Tetramethyl-Phenylendiamin (TMPD) |
| Enzym: | Glukoseoxidase (*Aspergillus niger*) |
| Schutzschicht: | Polyvinylpyrrolidon (PVP) |
| Ausführungsform | Die Reaktionsschicht besteht aus einer einzelnen Schicht enthaltend ein elektronenleitendes Material, den Mediator und die analyterkennende Biokomponente (Glukoseoxidase) |

TMPD-modifizierte Biosensoren ohne Schutzschicht weisen zu Beginn der Meßreihe im zyklischen Voltammogramm in glukosefreiem Puffer bei 100 mV einen Oxidationspeak mit einem Peakstrom von 90 nA (vs. Ag/AgCl) auf (Abbildung 9). Durch eine PVP-Schutzschicht läßt sich dieses Signal auf 850 nA verstärken (Abbildung 10). Innerhalb von 46 Tagen reduziert sich die oxidierbare TMPD-Menge der ungeschützten Biosensoren um 57%. Bei TMPD-Biosensoren mit PVP-Schutzschicht hingegen bleibt der Oxidationsstrom und damit die oxidierbare TMPD-Menge in diesem Zeitraum nahezu konstant.

TMPD zeigt in den zyklischen Voltammogrammen in gepufferter Glukoselösung ($c_{Glukose}$=15 mM) die Verwendbarkeit als Mediator. Der Strom im Potentialbereich der Oxidation von TMPD liegt anfänglich bei Biosensoren ohne Schutzschicht bei 450 nA (Abbildung 11), bei Biosensoren mit Schutzschicht bei 3500 nA (Abbildung 12). Dieser Wert kann auch nur bei Biosensoren mit PVP-Schutzschicht nahezu konstant gehalten werden. Ohne Schutzschicht sinkt der Oxidationsstrom innerhalb von 46 Tagen bis auf 160 nA ab. Die stabilisierende und signalerhöhende Wirkung der PVP-Schutzschicht wird auch bei der Betrachtung der Sensitivität deutlich. Diese steigt durch die PVP-Schutzschicht von 60 nA/mM (Abbildung 13) auf 341 nA/mM (Abbildung 14) an und kann über 46 Tage auf diesem Niveau konstant gehalten werden. Wie bereits beim ADPA-Biosensor zu beobachten ist, verlängert die PVP-Schutzschicht auch beim TMPD-Biosensor den linearen Meßbereich, so dass eine Glukosekonzentration von 4 mM noch erfaßt werden kann.

Beispiel 3

| | |
|---|---|
| Elektrodenmaterial: | Kohlenstoff-Paste |
| Mediator: | N,N,N',N'-Tetramethyl-Phenylendiamin (TMPD) |
| Enzym: | Glukoseoxidase (*Aspergillus niger*) |
| Schutzschicht: | Stärke |
| Ausführungsform | Die Reaktionsschicht besteht aus einer einzelnen Schicht enthaltend ein elektronenleitendes Material, den Mediator und die analyterkennende Biokomponente (Glukoseoxidase) |

Ein TMPD-Biosensor läßt sich auch durch eine Schutzschicht aus Stärke stabilisieren. Die Signalintensität im zyklischen Voltammogramm in gepufferter Glukoselösung steigt durch diese Schicht auf 175 nA (Abbildung 15) an (im Vergleich zu 90 nA ohne Schutzschicht, Abbildung 9) und bleibt über einen Zeitraum von 46 Tagen nahezu konstant. In einer gepufferten Glukoselösung ($c_{Glukose}$=15 mM) erhält man im Zeitraum von 46 Tagen Oxidationsströme zwischen 470 und 520 nA (Abbildung 16) im Vergleich zu 450 nA ohne Schutzschicht (Abbildung 11). Die signalerhöhende Wirkung der Stärke ist somit nicht so intensiv wie bei PVP, eine Stabilisierung läßt sich mit dieser Schutzschicht dennoch erreichen. Dies wird auch bei den Sensitivitätsuntersuchungen deutlich (Abbildung 17).

B) Biosensoren zur Bestimmung der Konzentrationen von sequenzspezifischen DNA-Einzelstrangfragmenten (Beispiel 4) oder von Antigenen in Probenflüssigkeiten (Beispiel 5)

Mit dem zuvor beschriebenen Sensoraufbau lassen sich neben Analyten, die durch spezifische Enzyme umgesetzt werden, auch DNA-Einzelstrangfragmente oder aber auch Antigene in Proben- oder Körperflüssigkeiten quantifizieren.

Beispiel 4

| | |
|---|---|
| Elektrodenmaterial: | Kohlenstoff-Paste |
| Mediator: | p-Aminodiphenylamin (ADPA) |
| Enzym: | Peroxidase |
| Biokomponente: | biotinylierte einzelsträngige DNA |
| Schutzschicht: | Stärke |
| Ausführungsform: | Die Reaktionsschicht besteht aus einer einzelnen Schicht enthaltend ein elektronenleitendes Material, den Mediator und die analyterkennende Biokomponente (DNA-Sonde). |

Die biotinylierte sequenzspezifische DNA-Sonde liegt über Avidin in der Reaktionsschicht immobilisiert vor (Abbildung 18). Die zu bestimmende Menge an komplementärem DNA-Strang ist mit Digoxigenin markiert. Nach Hybridisierung dieses komplementären DNA-Stranges an den immobilisierten DNA-Einzelstrang erfolgt der Nachweis

nach Zugabe eines Anti-Digoxigenin-Antikörper-Konjugates (Enzym-Konjugat). Die Peroxidase katalysiert die Reduktion von Wasserstoffperoxid (Substrat des Enzym-Konjugates) zu Wasser, wodurch die Peroxidase oxidiert wird. Die Regeneration der Peroxidase erfolgt durch den Mediator ADPA, der oxidierte Mediator wird anschließend an der Arbeitselektrode bei einem Potential von -150 mV (vs. Ag/AgCl) wieder reduziert. In der Abbildung 19 ist eine Kalibriergerade für den Nachweis eines 20mer Oligonukleotids dargestellt. Die Nachweisgrenze liegt bei 80 amol an DNA.

Beispiel 5

| | |
|---|---|
| Elektrodenmaterial: | Kohlenstoff-Paste |
| Mediator: | p-Aminodiphenylamin (ADPA) |
| Enzym: | Peroxidase |
| Biokomponente: | Fänger-Antikörper |
| Schutzschicht: | Stärke |
| Ausführungsform: | Die Reaktionsschicht besteht aus eine einzelnen Schicht enthaltend ein elektronenleitendes Material, den Mediator und die analyterkennende Biokomponente (FängerAntikörper). |

Der Fänger-Antikörper liegt entweder direkt immobilisiert vor oder wird über zuvor immobilisiertes Avidin (Abb. 20) auf der Oberfläche fixiert. Nach Anbindung des Analyten aus der zu untersuchenden Probe an den analytspezifischen Fänger-Antikörper erfolgt der Nachweis nach Zugabe eines zweiten analytspezifischen Antikörper-Peroxidase-Konjugates (Enzym-Konjugat). Die Peroxidase katalysiert die Reduktion von $H_2O_2$ zu $H_2O$, wodurch die Peroxidase oxidiert wird. Die Regeneration der Peroxidase erfolgt durch den Mediator ADPA; der oxidierte Mediator wird an der Arbeitselektrode (bei E=-150 mV vs. Ag/AgCl) wieder reduziert. In der Abbildung 21 ist am Beispiel des Interleukin-4 eine Kalibriergerade dargestellt. Die Nachweisgrenze liegt bei 200 amol an Interleukin-4.

**Patentansprüche**

1. Einwegbiosensor umfassend ein Trägermaterial (1), auf dem elektrische Leiterbahnen (2) sowie ein Elektrodensystem umfassend eine Gegenelektrode (6) und eine aus einer Reaktionsschicht gebildete Arbeitselektrode (5) aufgebracht sind, eine dielektrische Isolatorschicht (3), welche das Trägermaterial (1) sowie die elektrischen Leiterbahnen (2) bedeckt und Aussparungen für die Kontaktierung (4) einer Potentiostateinheit und für das Elektrodensystem aufweist, sowie eine analyterkennende Biokomponente, **dadurch gekennzeichnet, dass**

   - die Reaktionsschicht neben einem elektronenleitenden Material einen Elektronentransfermediator enthält, der einen Dampfdruck von mindestens $1{,}33 \cdot 10^{-3}$ $N/m^2$ ($1 \cdot 10^{-5}$ mmHg) bei 25 °C besitzt und

   - das Elektrodensystem von einer polymeren Schutzschicht (8) bedeckt ist.

2. Einwegbiosensor gemäß Anspruch 1, **dadurch gekennzeichnet, dass** der Elektronentransfermediator in der Reaktionsschicht eine Verbindung der Struktur (I) ist,

(I)

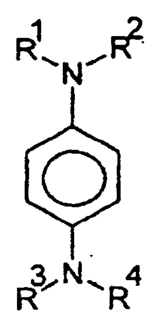

in der die Reste $R^1$, $R^2$, $R^3$ und $R^4$ gleich oder verschieden sein können und ein Wasserstoffatom, einen $C_1$-$C_{10}$-Alkylrest, bevorzugt einen $C_1$-$C_5$-Alkylrest, oder einen Arylrest darstellen.

3. Einwegbiosensor gemäß Anspruch 1 bis 2, **dadurch gekennzeichnet, dass** der Elektronentransfermediator in der Reaktionsschicht entweder p-Phenylendiamin, N,N,N',N'-Tetramethyl-p-Phenylendiamin (TMPD) oder p-Aminodiphenylamin (ADPA) ist.

4. Einwegbiosensor gemäß Anspruch 1 bis 3, **dadurch gekennzeichnet, dass** das Elektrodensystem zusätzlich eine Referenzelektrode (7) umfaßt.

5. Einwegbiosensor gemäß Anspruch 4, **dadurch gekennzeichnet, dass** die Referenzelektrode (7) aus einer als wesentliche Bestandteile Silberpartikel, Silberchlorid und ein leicht lösliches Chloridsalz enthaltenden Polymerpaste besteht.

6. Einwegbiosensor gemäß Anspruch 1 bis 5, **dadurch gekennzeichnet, dass** das Trägermaterial (1) aus Polycarbonat, Polyvinylchlorid (PVC), Polyethylenterephtalat (PET), Polypropylen, Polyester oder Polystyrol besteht.

7. Einwegbiosensor gemäß Anspruch 1 bis 6, **dadurch gekennzeichnet, dass** die Leiterbahnen (2) aus einer als wesentliche Bestandteile Kohlenstoff-, Silber-, Platin- oder Goldpartikel enthaltenden Polymerpaste bestehen.

8. Einwegbiosensor gemäß Anspruch 1 bis 7, **dadurch gekennzeichnet, dass** zur Herstellung der Reaktionsschicht eine Polymerpaste als elektronenleitendes Material verwendet worden ist, die als wesentliche Bestandteile Kohlenstoff-, Platin-, Palladium-, Rhodium- oder Goldpartikel enthält.

9. Einwegbiosensor gemäß Anspruch 1 bis 8, **dadurch gekennzeichnet, dass** die Gegenelektrode (6) aus einer als wesentliche Bestandteile Silberpartikel, Silberchlorid und ein leicht lösliches Silbersalz enthaltenden Polymerpaste besteht.

10. Einwegbiosensor gemäß Anspruch 1 bis 9, **dadurch gekennzeichnet, dass** die hydrophile, polymere Schutzschicht (8) im wesentlichen aus einem Hydrogel aus Polyvinylpyrrolidon (PVP), Polyethylenoxid (PEO), Stärke oder Gelatine besteht.

11. Einwegbiosensor gemäß Anspruch 1 bis 10, **dadurch gekennzeichnet, dass** über dem Elektrodensystem ein Kunststoffgewebe (9) lokalisiert ist, welches mit einem die polymere Schutzschicht (8) bedeckenden Tape (10) über den Elektroden fixiert ist, wobei das Tape (10) eine Aussparung zur Aufnahme einer Flüssigkeit aufweist.

12. Einwegbiosensor gemäß Anspruch 11, **dadurch gekennzeichnet, dass** das Kunststoffgewebe (9) aus einem Polyethylen-, Polypropylen- oder Polyamidnetz besteht.

13. Einwegbiosensor gemäß Anspruch 11, **dadurch gekennzeichnet, dass** das Tape (10) aus einer einseitig klebenden Polyester-, PVC- oder Papierklebefolie besteht.

14. Einwegbiosensor gemäß Anspruch 1 bis 13, **dadurch gekennzeichnet, dass** die analyterkennende Biokomponente ein Enzym ist.

15. Einwegbiosensor gemäß Anspruch 14, **dadurch gekennzeichnet, dass** das Enzym eine Oxidoreduktase, eine Dehydrogenase, eine β-Galaktosidase oder eine alkalische Phosphatase ist.

16. Einwegbiosensor gemäß Anspruch 14 bis 15, **dadurch gekennzeichnet, dass** das Enzym entweder Glukoseoxidase (GOD), Laktatoxidase (LOD), Uricase, Ascorbatoxidase, Ethanoloxidase, Cholesteroloxidase oder Peroxidase (POD) ist.

17. Einwegbiosensor gemäß Anspruch 1 bis 13, **dadurch gekennzeichnet, dass** die analyterkennende Biokomponente ein DNA-Molekül, ein Fänger-Antikörper oder ein Rezeptor ist.

18. Einwegbiosensor gemäß Anspruch 1 bis 17, **dadurch gekennzeichnet, dass** die Reaktionsschicht aus einer einzigen Schicht enthaltend die elektronenleitende Polymerpaste, den Elektronentransfermediator sowie die analyterkennende Biokomponente gebildet wird.

19. Einwegbiosensor gemäß Anspruch 1 bis 17, **dadurch gekennzeichnet, dass** die Reaktionsschicht aus einer einzigen Schicht enthaltend die elektronenleitende Polymerpaste und den Elektronentransfermediator gebildet wird

und dass die analyterkennende Biokomponente in der polymeren Schutzschicht (8) lokalisiert ist.

**20.** Einwegbiosensor gemäß Anspruch 1 bis 17, **dadurch gekennzeichnet, dass** die Reaktionsschicht aus zwei Schichten besteht, wobei die erste Schicht eine elektronenleitende Polymerpaste sowie einen Elektronentransfermediator enthält und von einer zweiten, hydrophilen polymeren Deckschicht bedeckt ist, die als Träger der analyterkennenden Biokomponente fungiert.

**21.** Einwegbiosensor gemäß Anspruch 1 bis 17, **dadurch gekennzeichnet, dass** die Reaktionsschicht aus zwei Schichten besteht, wobei die erste Schicht aus der elektronenleitenden Polymerpaste gebildet wird und von einer zweiten, hydrophilen polymeren Deckschicht bedeckt ist, die als Träger der analyterkennenden Biokomponente und des Elektronentransfermediators fungiert.

**22.** Einwegbiosensor gemäß Anspruch 1 bis 17, **dadurch gekennzeichnet, dass** die Reaktionsschicht aus zwei Schichten besteht, wobei die erste Schicht aus der elektronenleitenden Polymerpaste gebildet wird und von einer zweiten, hydrophilen polymeren Deckschicht bedeckt ist, die als Träger des Elektronentransfermediators fungiert und dass die analyterkennende Biokomponente in der polymeren Schutzschicht (8) lokalisiert ist.

**23.** Einwegbiosensor gemäß Anspruch 20 bis 22, **dadurch gekennzeichnet, dass** die hydrophile polymere Deckschicht im wesentlichen aus einem Hydrogel aus Polyvinylpyrrolidon (PVP), Polyethylenoxid (PEO), Stärke oder Gelatine besteht.

**24.** Verfahren zur Bestimmung eines Analyten in einer flüssigen Probe unter Verwendung des Biosensors gemäß Anspruch 14, **dadurch gekennzeichnet, dass** eine flüssige Probe auf das Elektrodensystem des Biosensors gebracht wird und anschließend über eine Potentiostateinheit, mit der ein definiertes Meßpotential vorgegeben wird, der zur Analytkonzentration proportionale Strom, welcher das Meßsignal des Biosensors darstellt, bestimmt wird.

**25.** Verfahren zur Bestimmung eines Analyten in einer flüssigen Probe unter Verwendung des Biosensors gemäß Anspruch 17, **dadurch gekennzeichnet, dass** eine flüssige Probe, welche eine sequenzspezifische DNA, ein Antigen oder einen Liganden enthält, auf das Elektrodensystem des Biosensors gebracht wird, danach der Sensor mit einer Pufferlösung gründlich gewaschen wird, anschließend die Elektroden zunächst mit einer Lösung enthaltend ein Enzym-Konjugat, welches die sequenzspezifsche DNA, das Antigen oder den Liganden erkennt, und nach erneutem Waschen mit einer Lösung enthaltend ein Substrat bedeckt werden und schließlich über eine Potentiostateinheit, mit der ein definiertes Meßpotential vorgegeben wird, der zur DNA-Konzentration, zur Antigen-Konzentration oder zur Ligand-Konzentration proportionale Strom, welcher das Meßsignal des Biosensors darstellt, bestimmt wird.

**26.** Verwendung von Verbindungen, die einen Dampfdruck von mindestens $1 \cdot 10^{-5}$ mmHg bei 25 °C besitzen als Elektronentransfermediatoren eines elektrochemischen Sensors zur Überführung von Elektronen von einem Enzym zu einem elektronenieitenden Material, wobei sich die Verbindung in einer sogenannten Reaktionsschicht befindet.

**27.** Verwendung von Verbindungen, die einen Dampfdruck von mindestens $1 \cdot 10^{-5}$ mmHg bei 25 °C besitzen als Elektronentransfermediatoren gemäß Anspruch 26, **dadurch gekennzeichnet, dass** die Verbindung eine Struktur gemäß der Formel (I) aufweist.

(I)

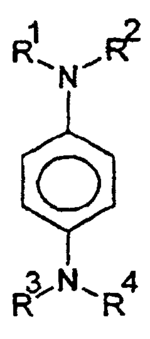

in der die Reste $R^1$, $R^2$, $R^3$ und $R^4$ gleich oder verschieden sein können und ein Wasserstoffatom, einen $C_1$-$C_{10}$-Alkylrest, bevorzugt einen $C_1$-$C_5$-Alkylrest, oder einen Arylrest darstellen.

**28.** Verwendung von Verbindungen, die einen Dampfdruck von mindestens $1 \cdot 10^{-5}$ mmHg bei 25 °C besitzen als Elektronentransfermediatoren gemäß Anspruch 26 und 27, **dadurch gekennzeichnet, dass** es sich bei der Verbindung um p-Phenylendiamin, N,N,N',N'-Tetramethyl-p-Phenylendiamin (TMPD) oder p-Aminodiphenylamin (ADPA) handelt.

**29.** Verwendung eines Einwegbiosensors gemäß 1 bis 23 zur "in-vitro" Bestimmung von Analytkonzentration in einer synthetischen Lösung, in einer Nährlösung oder in einer Körperflüssigkeit wie etwa Blut, Blutplasma, Blutserum oder Urin.

**30.** Verwendung eines Einwegbiosensors gemäß Anspruch 29 **dadurch gekennzeichnet**, das der Analyt Glukose, Laktat, Harnsäure, Ascorbat, Ethanol, Cholesterol oder Wasserstoffperoxid ist.

## Claims

**1.** A disposable biosensor comprising a support material

(1) onto which are applied electrically conductive tracks (2) and an electrode system comprising a counter-electrode (6) and a working electrode (5) formed from a reaction layer, a dielectric insulation layer (3), which covers the support material (1) and the electrically conductive tracks (2) and comprises recesses for contacting (4) a potentiostat unit and for the electrode system, together with an analyte-detecting biocomponent, **characterised in that**,

- in addition to an electron-conducting material, the reaction layer contains an electron-transfer mediator which has a vapour pressure of at least $1.33 \cdot 10^{-3}$ N/m$^2$ ($1 \cdot 10^{-5}$ mm Hg) at 25°C and
- the electrode system is covered by a polymeric protective layer (8).

**2.** A disposable biosensor according to claim 1, **characterised in that** the electron-transfer mediator in the reaction layer is a compound of the structure (I),

(I)

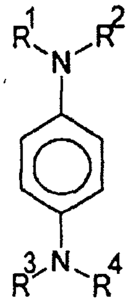

in which the residues $R^1$, $R^2$, $R^3$ and $R^4$ may be identical or different and represent a hydrogen atom, a $C_1$-$C_{10}$ alkyl residue, preferably a $C_1$-$C_5$ alkyl residue, or an aryl residue.

**3.** A disposable biosensor according to claims 1 to 2, **characterised in that** the electron-transfer mediator in the reaction layer is one of p-phenylenediamine, N,N,N',N'-tetramethyl-p-phenylenediamine (TMPD) or p-aminodiphenylamine (ADPA).

**4.** A disposable biosensor according to claims 1 to 3, **characterised in that** the electrode system additionally comprises a reference electrode (7).

**5.** A disposable biosensor according to claim 4, **characterised in that** the reference electrode (7) consists of a polymer paste containing silver particles, silver chloride and a readily soluble chloride salt as essential constituents.

6. A disposable biosensor according to claims 1 to 5, **characterised in that** the support material (1) consists of polycarbonate, polyvinyl chloride (PVC), polyethylene terephthalate (PET), polypropylene, polyester or polystyrene.

7. A disposable biosensor according to claims 1 to 6, **characterised in that** the conductive tracks (2) consist of a polymer paste containing carbon, silver, platinum or gold particles as essential constituents.

8. A disposable biosensor according to claims 1 to 7, **characterised in that** the reaction layer has been produced using as the electron-conducting material a polymer paste which contains carbon, platinum, palladium, rhodium or gold particles as essential constituents.

9. A disposable biosensor according to claims 1 to 8, **characterised in that** the counter-electrode (6) consists of a polymer paste containing silver particles, silver chloride and a readily soluble silver salt as essential constituents.

10. A disposable biosensor according to claims 1 to 9, **characterised in that** the hydrophilic, polymeric protective layer (8) substantially consists of a hydrogel of polyvinylpyrrolidone (PVP), polyethylene oxide (PEO), starch or gelatine.

11. A disposable biosensor according to claims 1 to 10, **characterised in that** a woven plastics fabric (9) is located over the electrode system, which fabric is fixed over the electrodes with a tape (10) covering the polymeric protective layer (8), wherein the tape (10) comprises a recess for accommodating a liquid.

12. A disposable biosensor according to claim 11, **characterised in that** the woven plastics fabric (9) consists of a polyethylene, polypropylene or polyamide mesh.

13. A disposable biosensor according to claim 11, **characterised in that** the tape (10) consists of a single-sided adhesive polyester, PVC or paper sheet.

14. A disposable biosensor according to claims 1 to 13, **characterised in that**, the analyte-detecting biocomponent is an enzyme.

15. A disposable biosensor according to claim 14, **characterised in that** the enzyme is an oxidoreductase, a dehydrogenase, a β-galactosidase or an alkaline phosphatase.

16. A disposable biosensor according to claims 14 to 15, **characterised in that** the enzyme is one of glucose oxidase (GOD), lactate oxidase (LOD), uricase, ascorbate oxidase, ethanol oxidase, cholesterol oxidase or peroxidase (POD).

17. A disposable biosensor according to claims 1 to 13, **characterised in that** the analyte-detecting biocomponent is a DNA molecule, a scavenger antibody or a receptor.

18. A disposable biosensor according to claims 1 to 17, **characterised in that** the reaction layer is formed from a single layer containing the electron-conducting polymer paste, the electron-transfer mediator and the analyte-detecting biocomponent.

19. A disposable biosensor according to claims 1 to 17, **characterised in that** the reaction layer is formed from a single layer containing the electron-conducting polymer paste and the electron-transfer mediator and that the analyte-detecting biocomponent is located in the polymeric protective layer (8).

20. A disposable biosensor according to claims 1 to 17, **characterised in that** the reaction layer consists of two layers, wherein the first layer contains an electron-conducting polymer paste and an electron-transfer mediator and is covered by a second, hydrophilic polymeric outer layer, which acts as a carrier for the analyte-detecting biocomponent.

21. A disposable biosensor according to claims 1 to 17, **characterised in that** the reaction layer consists of two layers, wherein the first layer is formed from the electron-conducting polymer paste and is covered by a second, hydrophilic polymeric outer layer, which acts as a carrier for the analyte-detecting biocomponent and for the electron-transfer mediator.

**22.** A disposable biosensor according to claims 1 to 17, **characterised in that** the reaction layer consists of two layers, wherein the first layer is formed from the electron-conducting polymer paste and is covered by a second, hydrophilic polymeric outer layer, which acts as a carrier for the electron-transfer mediator and that the analyte-detecting biocomponent is located in the polymeric protective layer (8).

**23.** A disposable biosensor according to claims 20 to 22, **characterised in that** the hydrophilic, polymeric outer layer substantially consists of a hydrogel of polyvinylpyrrolidone (PVP), polyethylene oxide (PEO), starch or gelatine.

**24.** A method for determining an analyte in a liquid sample using the biosensor according to claim 14, **characterised in that** a liquid sample is placed on the electrode system of the biosensor and then the current, which is proportional to the analyte concentration and constitutes the measurement signal of the biosensor, is determined by means of a potentiostat unit, which sets a defined measuring potential.

**25.** A method for determining an analyte in a liquid sample using the biosensor according to claim 17, **characterised in that** a liquid sample, which contains sequence-specific DNA, an antigen or a ligand, is placed on the electrode system of the biosensor, then the sensor is thoroughly washed with a buffer solution, after which the electrodes are covered firstly with a solution containing an enzyme conjugate, which detects the sequence-specific DNA, the antigen or the ligand, and, after washing again, then with a solution containing a substrate and finally the current, which is proportional to the DNA concentration, the antigen concentration or the ligand concentration and constitutes the measurement signal of the biosensor, is determined by means of a potentiostat unit, which sets a defined measuring potential.

**26.** Use of compounds which have a vapour pressure of at least $1 \cdot 10^{-5}$ mm Hg at 25°C as electron-transfer mediators of an electrochemical sensor for transferring electrons from an enzyme to an electron-conducting material, wherein the compound is located in a "reaction layer".

**27.** Use of compounds which have a vapour pressure of at least $1 \cdot 10^{-5}$ mm Hg at 25°C as electron-transfer mediators according to claim 26, **characterised in that** the compound exhibits a structure according to the formula (I),

(I) 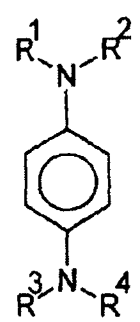

in which the residues $R^1$, $R^2$, $R^3$ and $R^4$ may be identical or different and represent a hydrogen atom, a $C_1$-$C_{10}$ alkyl residue, preferably a $C_1$-$C_5$ alkyl residue, or an aryl residue.

**28.** Use of compounds which have a vapour pressure of at least $1 \cdot 10^{-5}$ mm Hg at 25°C as electron-transfer mediators according to claims 26 and 27, **characterised in that** the compound comprises p-phenylenediamine, N,N,N',N'-tetramethyl-p-phenylenediamine (TMPD) or p-aminodiphenylamine (ADPA).

**29.** Use of a disposable biosensor according to claims 1 to 23 for the in vitro determination of analyte concentration in a synthetic solution, in a nutrient solution or in a bodily fluid such as for instance blood, blood plasma, blood serum or urine.

**30.** Use of a disposable biosensor according to claim 29, **characterised in that** the analyte is glucose, lactate, uric acid, ascorbate, ethanol, cholesterol or hydrogen peroxide.

**Revendications**

1. Biocapteur à usage unique, qui comprend un matériau de support (1) sur lequel sont appliquées des pistes électriquement conductrices (2) ainsi qu'un système d'électrodes qui comprend une contre-électrode (6) et une électrode de travail (5) formées d'une couche de réaction, une couche diélectrique d'isolant (3) qui recouvre le matériau de support (1) ainsi que les pistes électriquement conductrices (2) et qui présente des découpes pour la mise en contact (4) d'une unité de potentiostat et pour le système d'électrodes, ainsi qu'un biocomposant de détection d'un analyte, **caractérisé en ce que**

   - la couche de réaction contient en plus d'un matériau conducteur d'électrons un médiateur de transfert d'électrons qui possède une tension de vapeur d'au moins $1{,}33 \cdot 10^{-3}$ N/m$^2$ ($1 \cdot 10^{-5}$ mm Hg) à 25°C et
   - le système d'électrodes est recouvert par une couche polymère de protection (8).

2. Biocapteur à usage unique selon la revendication 1, **caractérisé en ce que** le médiateur de transfert d'électrons prévu dans la couche de réaction est un composé de structure (I)

(I)

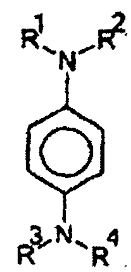

dans laquelle les groupes $R^1$, $R^2$, $R^3$ et $R^4$ peuvent être identiques ou différents et représentent un atome d'hydrogène, un groupe alkyle en $C_1$ à $C_{10}$, de préférence un groupe alkyle en $C_1$ à $C_5$, ou un groupe aryle.

3. Biocapteur à usage unique selon les revendications 1 à 2, **caractérisé en ce que** le médiateur de transfert d'électrons prévu dans la couche de réaction est soit la p-phénylènediamine, la N,N,N',N'-tétraméthyl-p-phénylènediamine (TMPD) ou la p-aminodiphénylamine (ADPA).

4. Biocapteur à usage unique selon les revendications 1 à 3, **caractérisé en ce que** le système d'électrodes comprend en outre une électrode de référence (7).

5. Biocapteur à usage unique selon la revendication 4, **caractérisé en ce que** l'électrode de référence (7) est constituée d'une pâte polymère qui contient comme composants essentiels des particules d'argent, du chlorure d'argent et un sel de chlorure facilement soluble.

6. Biocapteur à usage unique selon les revendications 1 à 5, **caractérisé en ce que** le matériau de support (1) est constitué de polycarbonate, de poly(chlorure de vinyle) (PVC), de poly(téréphtalate d'éthylène) (PET), de polypropylène, de polyester ou de polystyrène.

7. Biocapteur à usage unique selon les revendications 1 à 6, **caractérisé en ce que** les pistes conductrices (2) sont constituées d'une pâte polymère qui contient comme composants essentiels des particules de carbone, d'argent, de platine ou d'or.

8. Biocapteur à usage unique selon les revendications 1 à 7, **caractérisé en ce que** pour la réalisation de la couche de réaction, on utilise comme matériau conducteur d'électrons une pâte polymère qui contient comme composants essentiels des particules de carbone, de platine, de palladium, de rhodium ou d'or.

9. Biocapteur à usage unique selon les revendications 1 à 8, **caractérisé en ce que** la contre-électrode (6) est constituée d'une pâte polymère qui contient comme composants essentiels des particules d'argent, du chlorure d'argent et un sel d'argent facilement soluble.

**10.** Biocapteur à usage unique selon les revendications 1 à 9, **caractérisé en ce que** la couche polymère de protection (8) hydrophile est essentiellement constituée d'un hydrogel de polyvinylpyrrolidone (PVP), de poly(oxyde d'éthylène) (PEO), d'amidon ou de gélatine.

**11.** Biocapteur à usage unique selon les revendications 1 à 10, **caractérisé en ce qu'**un tissu (9) en matière synthétique, qui est fixé au-dessus des électrodes par un ruban (10) qui recouvre la couche polymère de protection (8), est localisé au-dessus du système d'électrodes, le ruban (10) présentant une découpe de réception d'un liquide.

**12.** Biocapteur à usage unique selon la revendication 11, **caractérisé en ce que** le tissu (9) en matière synthétique est constitué d'un réseau de polyéthylène, de polypropylène ou de polyamide.

**13.** Biocapteur à usage unique selon la revendication 11, **caractérisé en ce que** le ruban (10) est constitué d'une feuille adhésive en polyester, en PVC ou en papier adhésif sur une face.

**14.** Biocapteur à usage unique selon les revendications 1 à 13, **caractérisé en ce que** le biocomposant de détection d'analyte est une enzyme.

**15.** Biocapteur à usage unique selon la revendication 14, **caractérisé en ce que** l'enzyme est une oxydoréductase, une déshydrogénase, une β-galactosidase ou une phosphatase alcaline.

**16.** Biocapteur à usage unique selon les revendications 14 à 15, **caractérisé en ce que** l'enzyme est la glucosoxydase (GOD), la lactatoxydase (LOD), l'uricase, l'ascobatoxydase, l'éthanoloxydaxe, la cholestéroloxydase ou la peroxydase (POD).

**17.** Biocapteur à usage unique selon les revendications 1 à 13, **caractérisé en ce que** le biocomposant de détection d'analyte est une molécule d'ADN, un anticorps de piégeage ou un récepteur.

**18.** Biocapteur à usage unique selon les revendications 1 à 17, **caractérisé en ce que** la couche de réaction est formée d'une unique couche qui contient la pâte polymère conductrice d'électrons, le médiateur de transfert d'électrons ainsi que le biocomposant de détection d'analyte.

**19.** Biocapteur à usage unique selon les revendications 1 à 17, **caractérisé en ce que** la couche de réaction est formée d'une seule couche qui contient la pâte polymère conductrice d'électrons et le médiateur de transfert d'électrons et **en ce que** le biocomposant de détection d'analyte est localisé dans la couche polymère de protection (8).

**20.** Biocapteur à usage unique selon les revendications 1 à 17, **caractérisé en ce que** la couche de réaction est constituée de deux couches, la première couche contenant une pâte polymère conductrice d'électrons ainsi qu'un médiateur de transfert d'électrons et étant recouverte par une deuxième couche polymère hydrophile de recouvrement qui fonctionne comme support pour le biocomposant de détection d'analyte.

**21.** Biocapteur à usage unique selon les revendications 1 à 17, **caractérisé en ce que** la couche de réaction est constituée de deux couches, la première couche étant formée par la pâte polymère conductrice d'électrons et étant recouverte par une deuxième couche polymère hydrophile de recouvrement qui sert de support pour le biocomposant de détection d'analyte et le médiateur de transfert d'électrons.

**22.** Biocapteur à usage unique selon les revendications 1 à 17, **caractérisé en ce que** la couche de réaction est constituée de deux couches, la première couche étant formée par la pâte polymère conductrice d'électrons et étant recouverte par une deuxième couche polymère hydrophile de recouvrement qui joue le rôle d'un support pour le médiateur de transfert d'électrons et **en ce que** le biocomposant de détection d'analyte est localisé dans la couche polymère de protection (8).

**23.** Biocapteur à usage unique selon les revendications 20 à 22, **caractérisé en ce que** la couche polymère hydrophile de recouvrement est essentiellement constituée d'un hydrogel de polyvinylpyrrolidone (PVP), de poly(oxyde d'éthylène) (PEO), d'amidon ou de gélatine.

**24.** Procédé de détermination d'un analyte dans un échantillon liquide par recours au biocapteur selon la revendication 14, **caractérisé en ce qu'**un échantillon liquide est appliqué sur le système d'électrodes du biocapteur et qu'ensuite le courant qui est proportionnel à la concentration en analyte et qui représente le signal de mesure du biocapteur

est déterminé par une unité à potentiostat qui délivre un potentiel de mesure défini.

**25.** Procédé de détermination d'un analyte dans un échantillon liquide par recours au biocapteur selon la revendication 17, **caractérisé en ce qu'**un échantillon liquide qui contient un ADN à séquence spécifique, un antigène ou un ligand est appliqué sur le système d'électrodes du biocapteur, le capteur étant ensuite lavé à fond à l'aide d'une solution tampon, les électrodes étant ensuite lavées à l'aide d'une solution qui contient un conjugué d'enzyme qui reconnaît l'ADN de séquence spécifique, l'antigène ou le ligand, et après un nouveau lavage, sont recouvertes d'une solution qui contient un substrat et ensuite, le courant qui est proportionnel à la concentration en ADN, à la concentration en antigène ou à la concentration en ligand et qui constitue le signal de mesure du biocapteur est déterminé à l'aide d'une unité à potentiostat qui délivre un potentiel de mesure défini.

**26.** Utilisation de composés qui possèdent une tension de vapeur d'au moins $1{,}33 \cdot 10^{-3}$ $N/m^2$ ($1 \cdot 10^{-5}$ mm Hg) à 25°C comme médiateurs de transfert d'électrons d'un capteur électrochimique en vue du transfert d'électrons d'enzyme à un matériau conducteur d'électrons, le composé se trouvant dans une couche dite de réaction.

**27.** Utilisation de composés qui possèdent une tension de vapeur,d'au moins $1{,}33 \cdot 10^{-3}$ $N/m^2$ ($1 \cdot 10^{-5}$ mm Hg) à 25°C comme médiateurs de transfert d'électrons selon la revendication 26, **caractérisée en ce que** le composé présente une structure selon la formule (I)

(I)

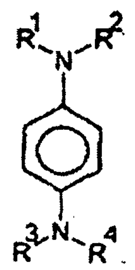

dans laquelle les groupes $R^1$, $R^2$, $R^3$ et $R^4$ peuvent être identiques ou différents et représentent un atome d'hydrogène, un groupe alkyle en $C_1$ à $C_{10}$, de préférence un groupe alkyle en $C_1$ à $C_5$, ou un groupe aryle.

**28.** Utilisation de composés qui possèdent une tension de vapeur d'au moins $1{,}33 \cdot 10^{-3}$ $N/m^2$ ($1 \cdot 10^{-5}$ mm Hg) à 25°C comme médiateurs de transfert d'électrons selon les revendications 26 et 27, **caractérisée en ce que** le composé est la p-phénylènediamine, la N,N,N',N'-tétraméthyl-p-phénylènediamine (TMPD) ou la p-aminodiphénylamine (ADPA).

**29.** Utilisation d'un capteur à usage unique selon les revendications 1 à 23 pour la détermination "in-vitro" de la concentration d'un analyte dans une solution synthétique, une solution nutritive ou dans un liquide corporel, par exemple le sang, le plasma sanguin, le sérum sanguin ou l'urine.

**30.** Utilisation d'un capteur à usage unique selon la revendication 29, **caractérisée en ce que** l'analyte est le glucose, un lactate, l'acide urique, un ascorbate, l'éthanol, le cholestérol ou le peroxyde d'hydrogène.

Abbildung 1:

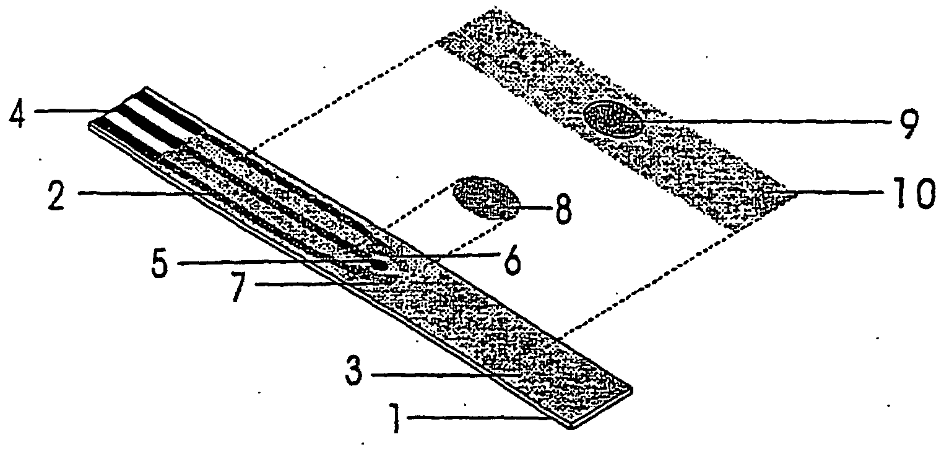


Abbildung 2:

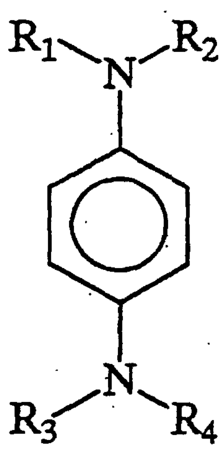

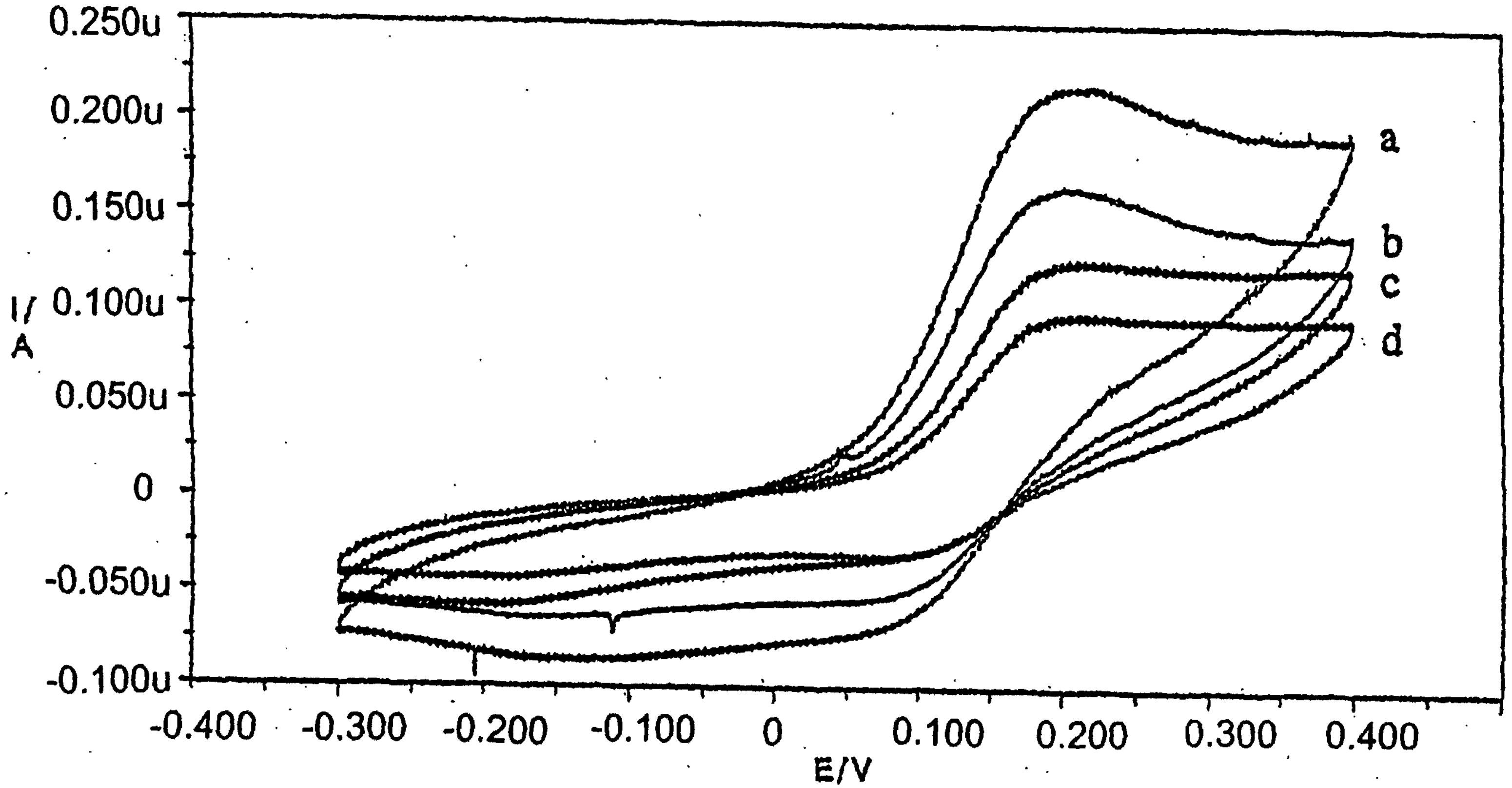


Abbildung 3: CV der ADPA-modifizierten Glukosebiosensoren ohne Schutzschicht, gemessen an unterschiedlichen Tagen, in glukosefreiem PBS-Puffer, (pH 7.4 ; $c_{(Cl^-)}$ 154 mM), v = 50 mV/s

a: 1. Tag ; b: 8. Tag ; c: 17. Tag ; d: 54. Tag

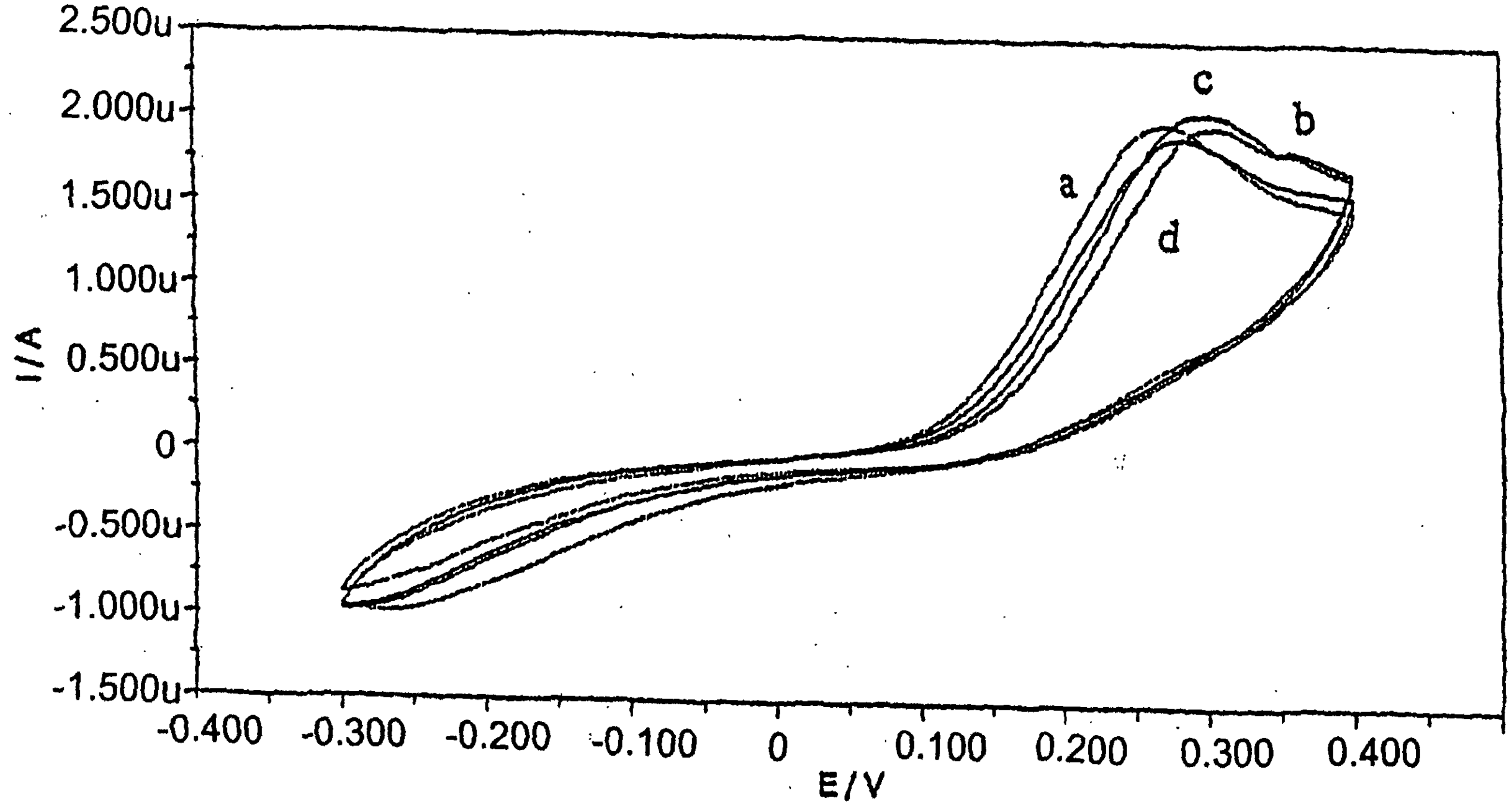


Abbildung 4: CV der ADPA-modifizierten Glukosebiosensoren mit PVP-Schutzschicht, gemessen an unterschiedlichen Tagen, in glukosefreiem PBS-Puffer, (pH 7.4 ; $c_{(Cl^-)}$ 154 mM), v = 50 mV/s

a: 1. Tag; b: 8. Tag; c: 17. Tag; d: 54. Tag

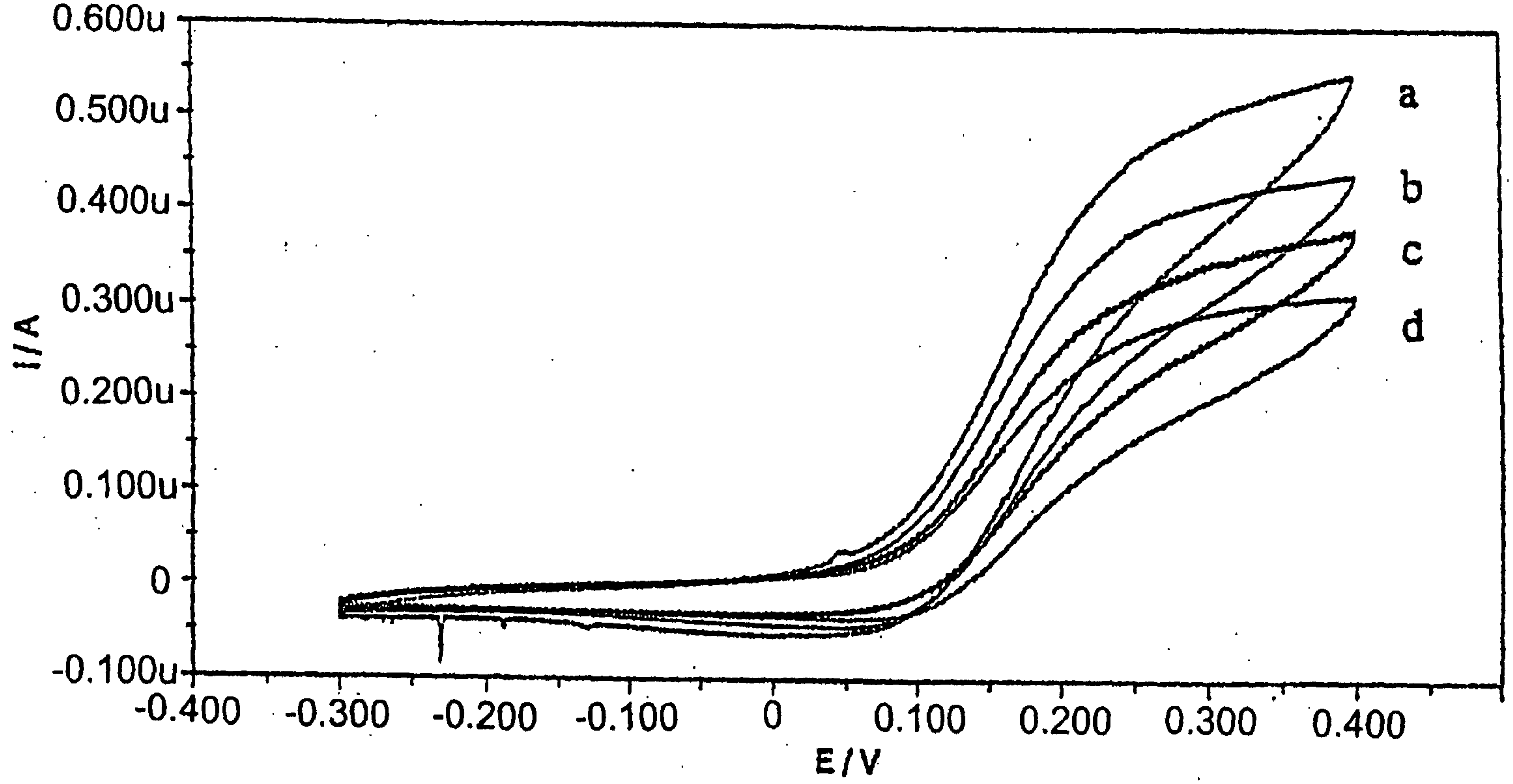


Abbildung 5: CV der ADPA-modifizierten Glukosebiosensoren ohne Schutzschicht, gemessen an unterschiedlichen Tagen, in gepufferter Glukoselösung, (pH 7.4; $c_{(Glukose)}$: 15 mM, $c_{(Cl^-)}$: 154 mM), v = 50 mV/s

a: 1. Tag; b: 8. Tag; c: 17. Tag; d: 54. Tag

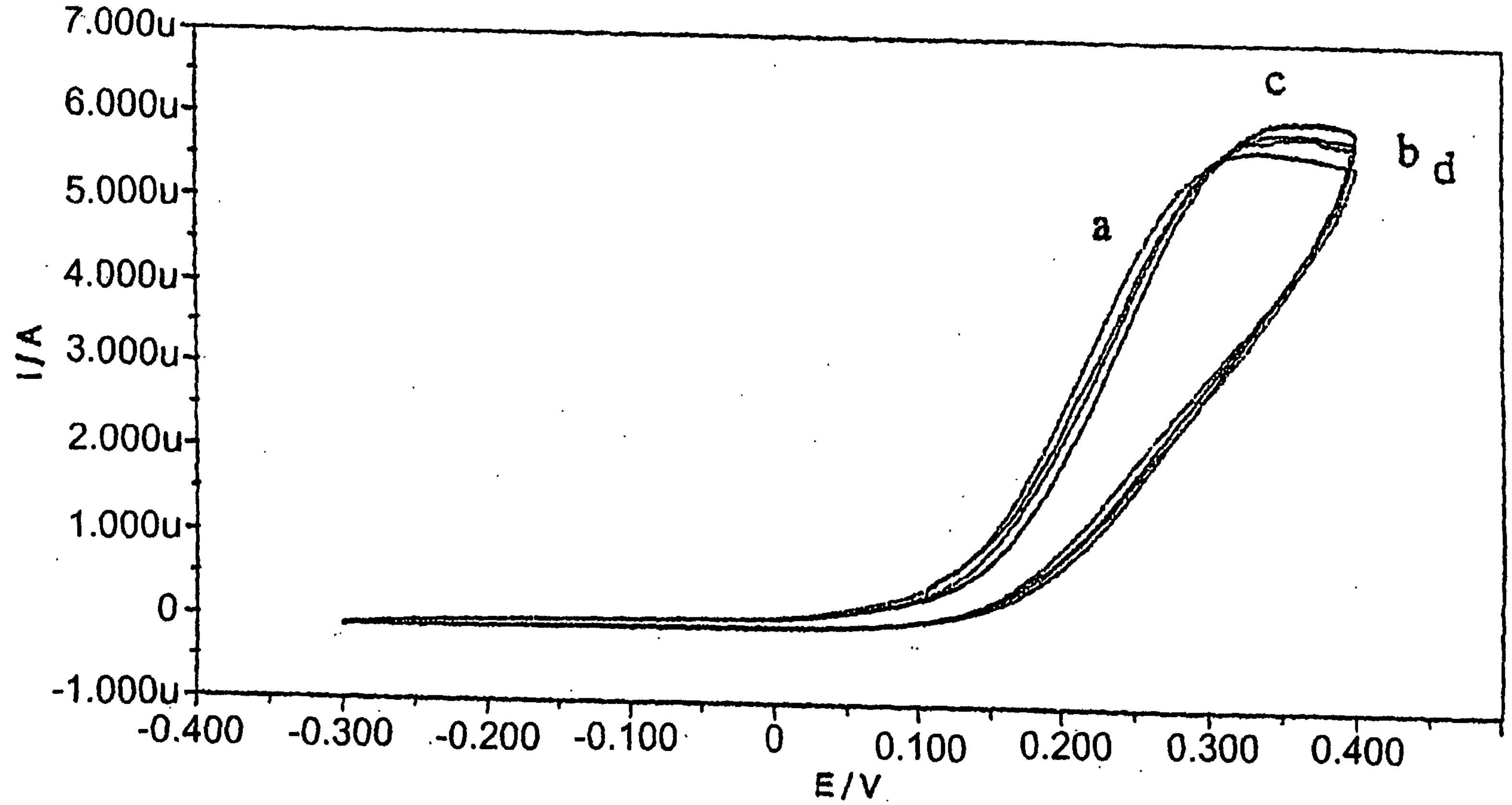


Abbildung 6: CV der ADPA-modifizierten Glukosebiosensoren mit PVP-Schutzschicht, gemessen an unterschiedlichen Tagen, in gepufferter Glukoselösung, (pH 7.4 ; $c_{(Glukose)}$: 15 mM, $c_{(Cl^-)}$: 154 mM), v = 50 mV/s

a: 1. Tag; b: 8. Tag; c: 17. Tag; d: 54. Tag

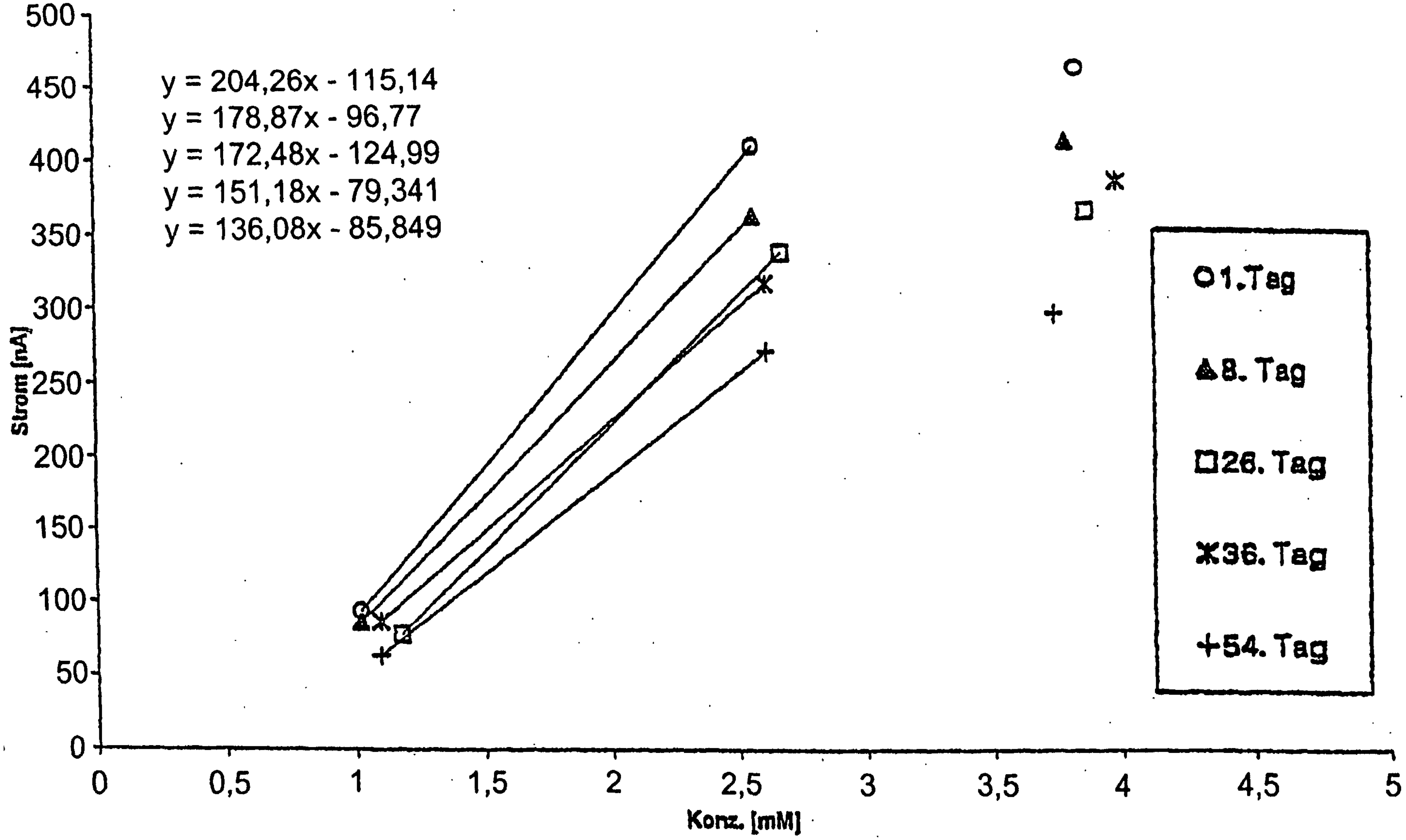


Abbildung 7: Sensitivität von ADPA-modifizierten Glukosebiosensoren ohne Schutzschicht in Abhängigkeit des Sensoralters

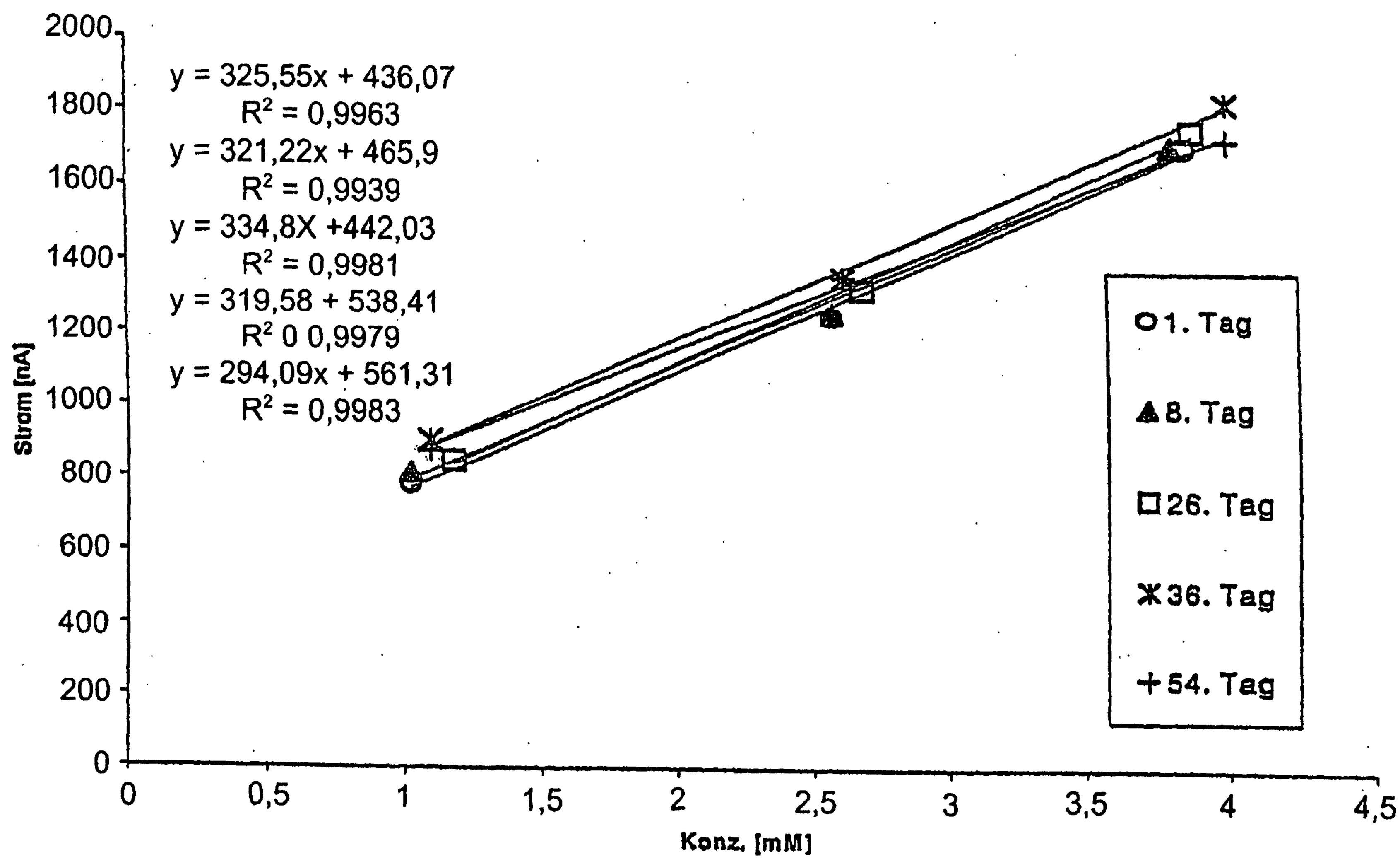


Abbildung 8: Sensitivität von ADPA-modifizierten Glukosebiosensoren mit PVP-Schutzschicht in Abhängigkeit des Sensoralters

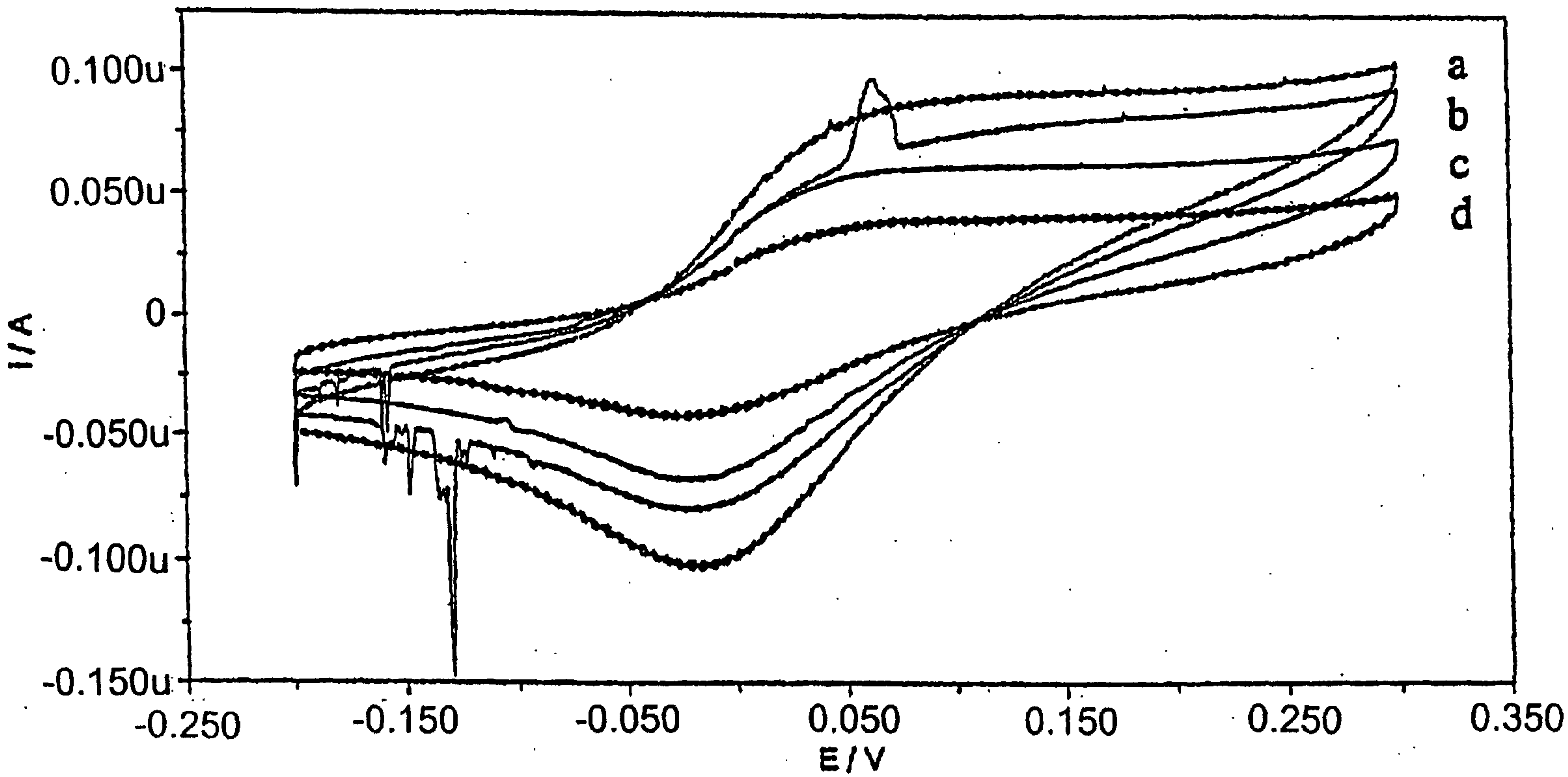


Abbildung 9: CV der TMPD-modifizierten Glukosebiosensoren ohne Schutzschicht, gemessen an unterschiedlichen Tagen, in glukosefreiem PBS-Puffer, (pH 7.4 ; $c_{(Cl^-)}$ 154 mM), v = 50 mV/s

a: 1. Tag ; b: 9. Tag ; c: 18. Tag ; d: 46. Tag

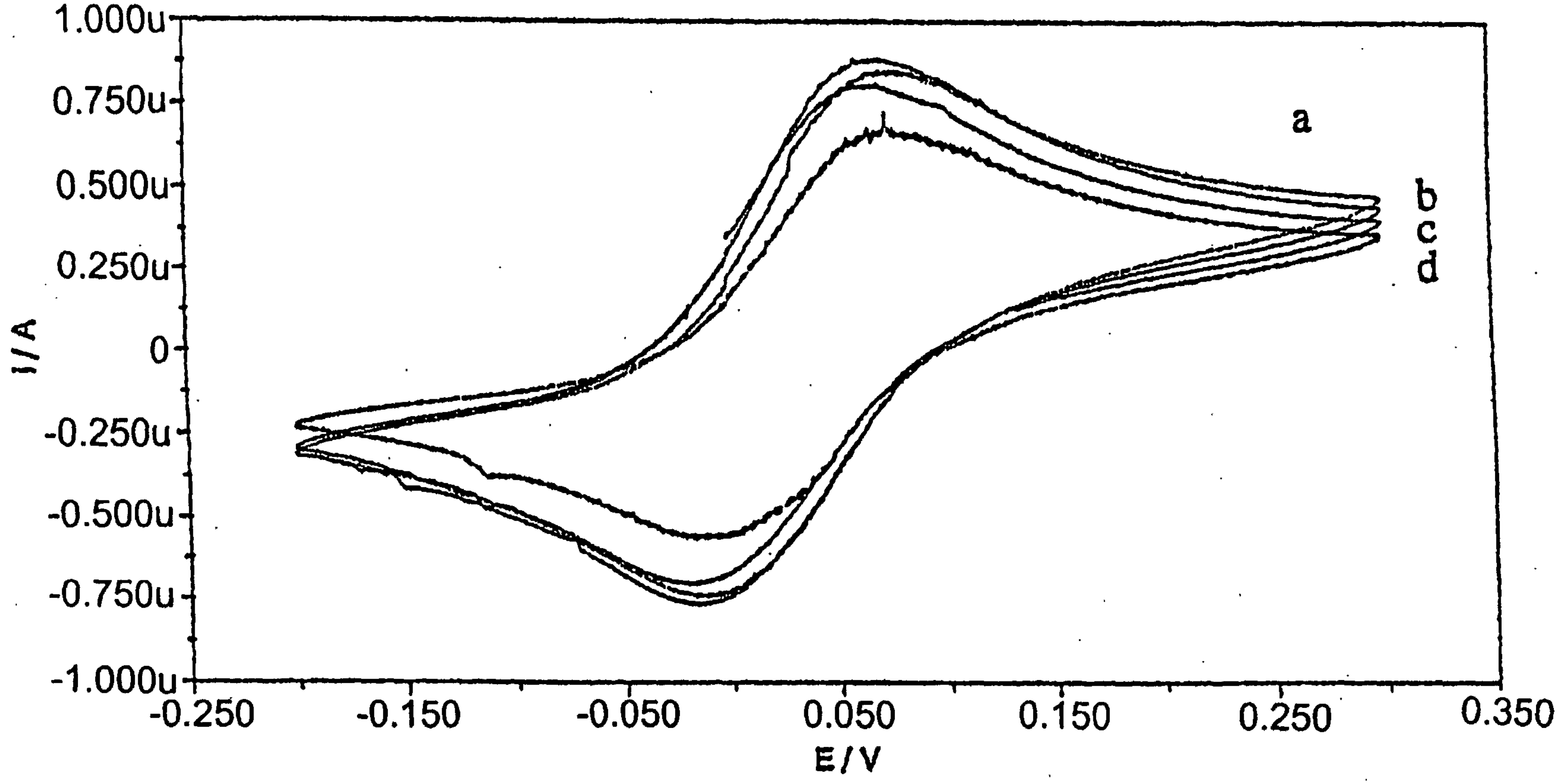


Abbildung 10: CV der TMPD-modifizierten Glukosebiosensoren mit PVP-Schutzschicht, gemessen an unterschiedlichen Tagen, in glukosefreiem PBS-Puffer, (pH 7.4 ; $c_{(Cl^-)}$ 154 mM), v = 50 mV/s

a: 1. Tag ; b: 9. Tag ; c: 18. Tag ; d: 46. Tag

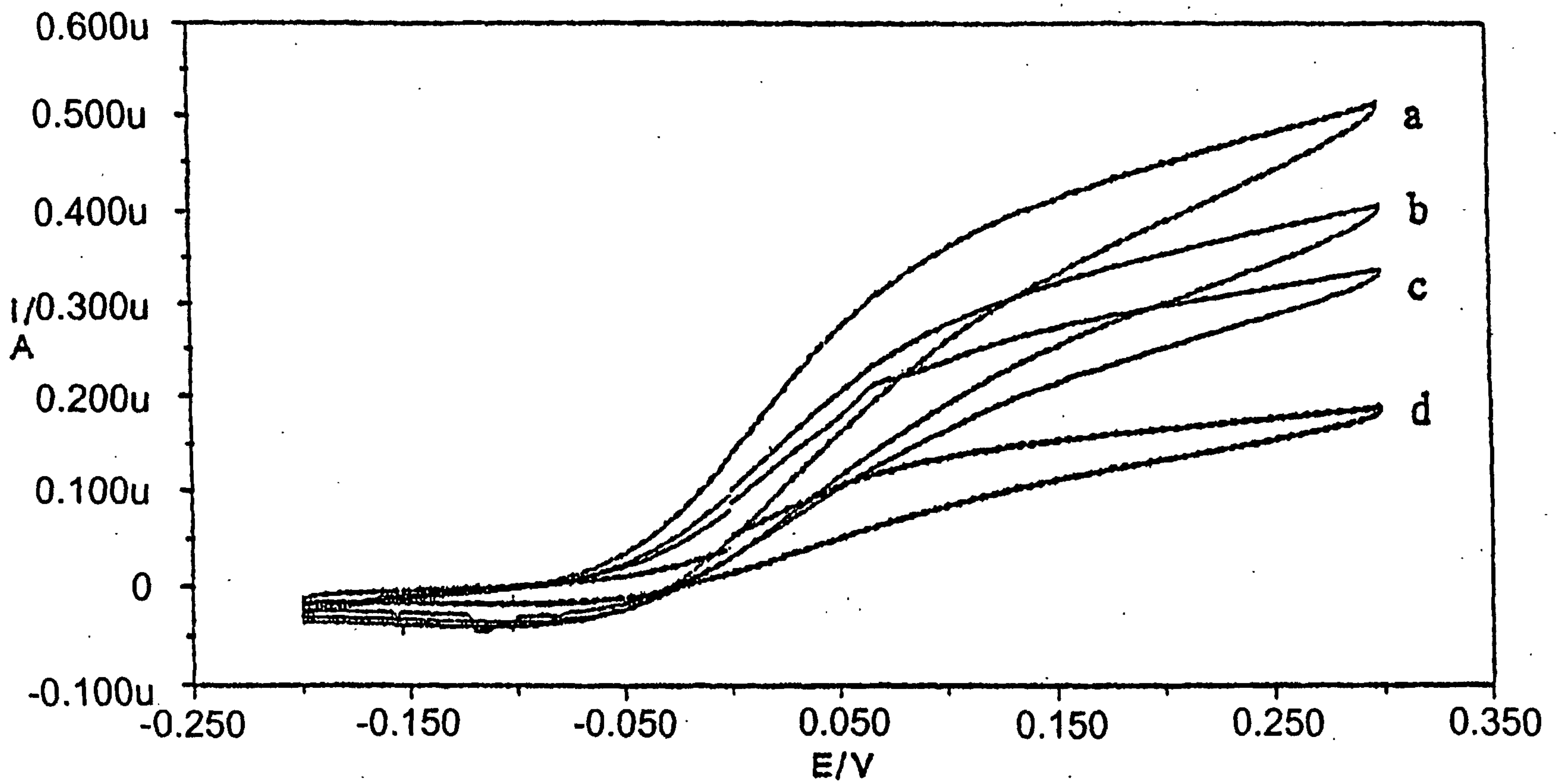


Abbildung 11: CV der TMPD-modifizierten Glukosebiosensoren ohne Schutzschicht, gemessen an unterschiedlichen Tagen, in gepufferter Glukoselösung, (pH 7.4 ; $c_{(Glukose)}$: 15 mM, $c_{(Cl^-)}$: 154 mM), v = 50 mV/s

a: 1. Tag ; b: 9. Tag ; c: 18. Tag ; d: 46. Tag

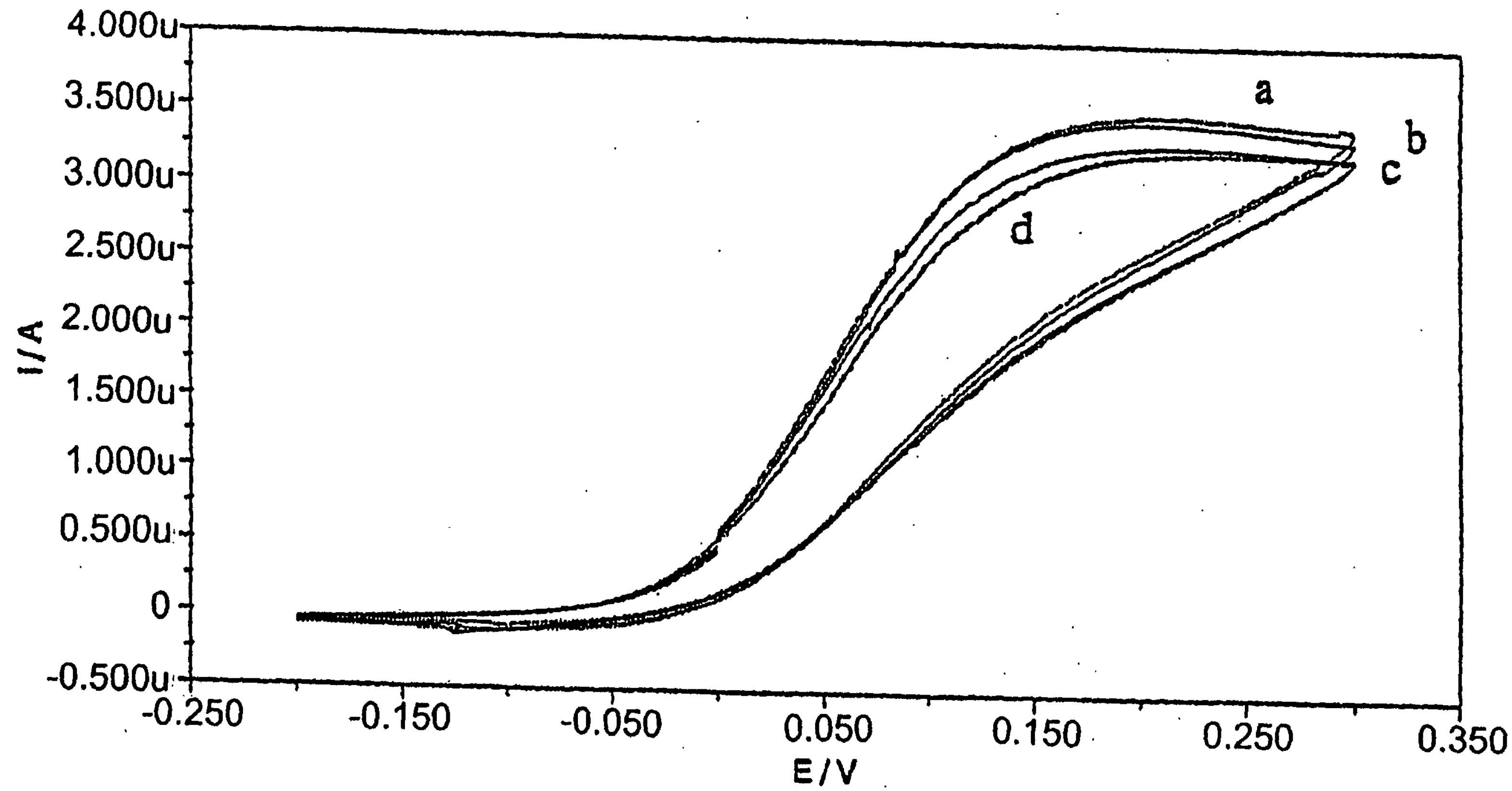


Abbildung 12: CV der TMPD-modifizierten Glukosebiosensoren mit PVP-Schutzschicht, gemessen an unterschiedlichen Tagen, in gepufferter Glukoselösung, (pH 7.4 ; $c_{(Glukose)}$: 15 mM, $c_{(Cl^-)}$: 154 mM), v = 50 mV/s

a: 1. Tag ; b: 9. Tag ; c: 18. Tag ; d: 46. Tag

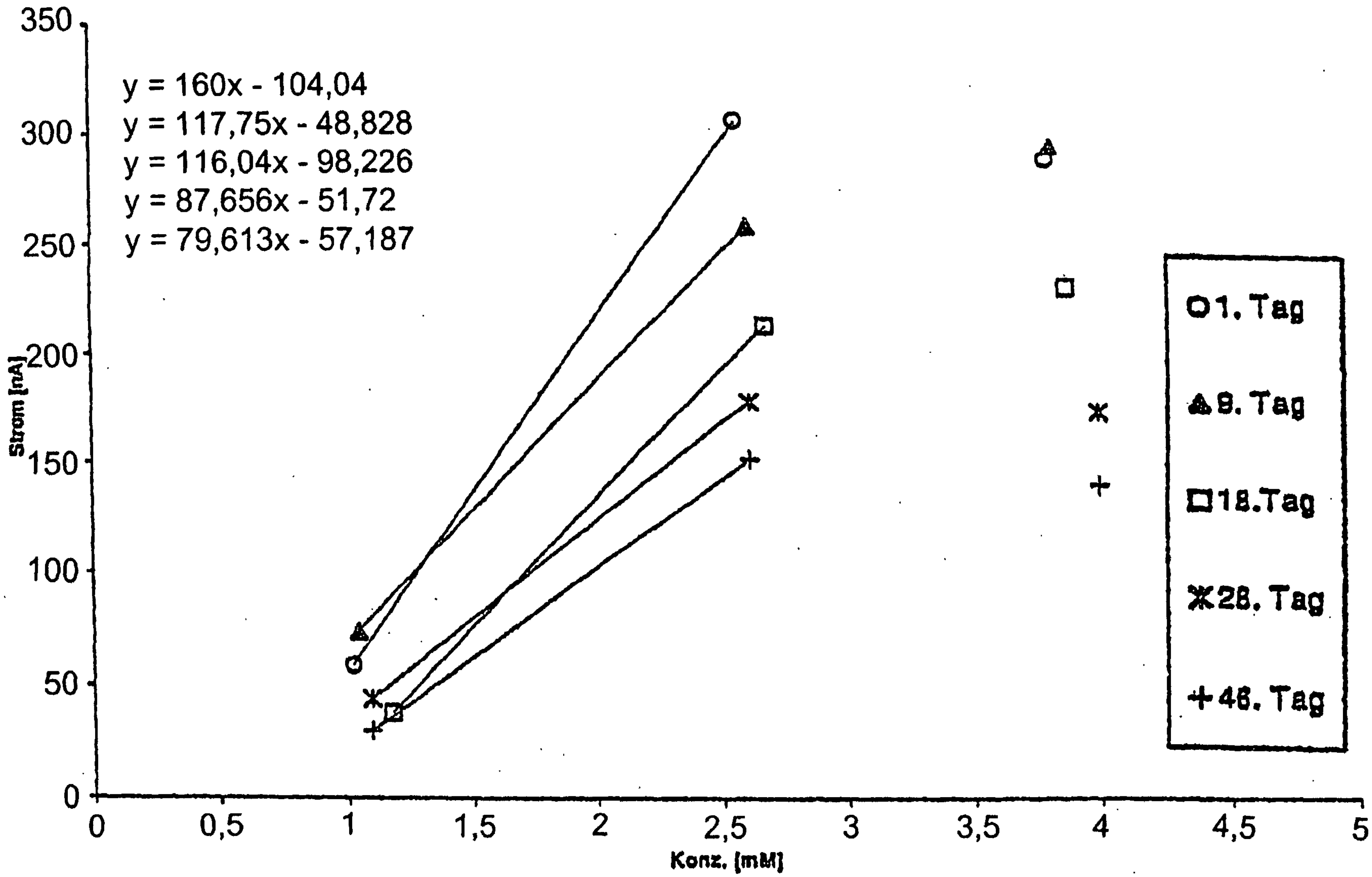


Abbildung 13: Sensitivität von TMPD-modifizierten Glukosebiosensoren ohne Schutzschicht in Abhängigkeit des Sensoralters

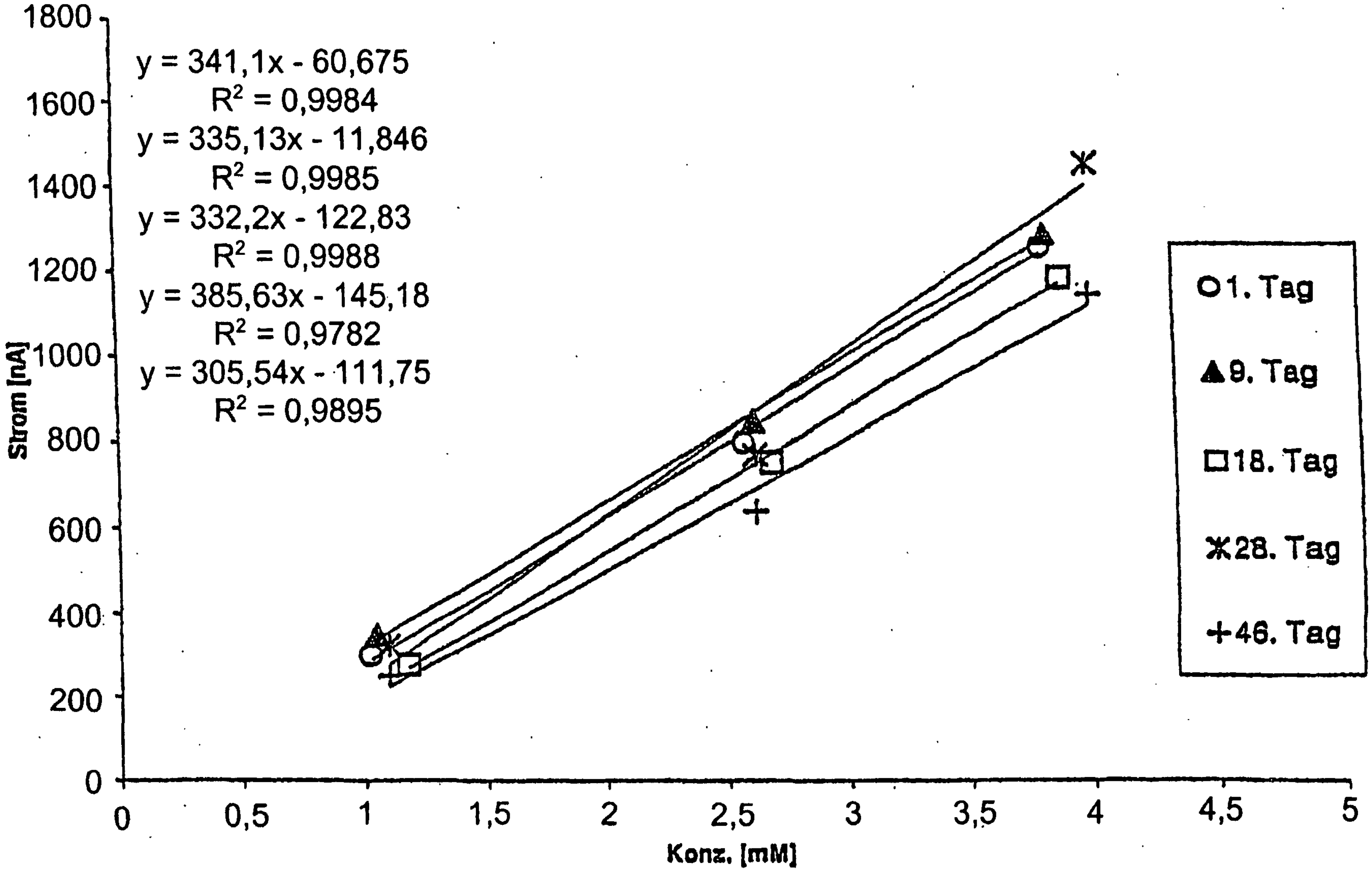


Abbildung 14: Sensitivität von TMPD-modifizierten Glukosebiosensoren mit PVP-Schutzschicht in Abhängigkeit des Sensoralters

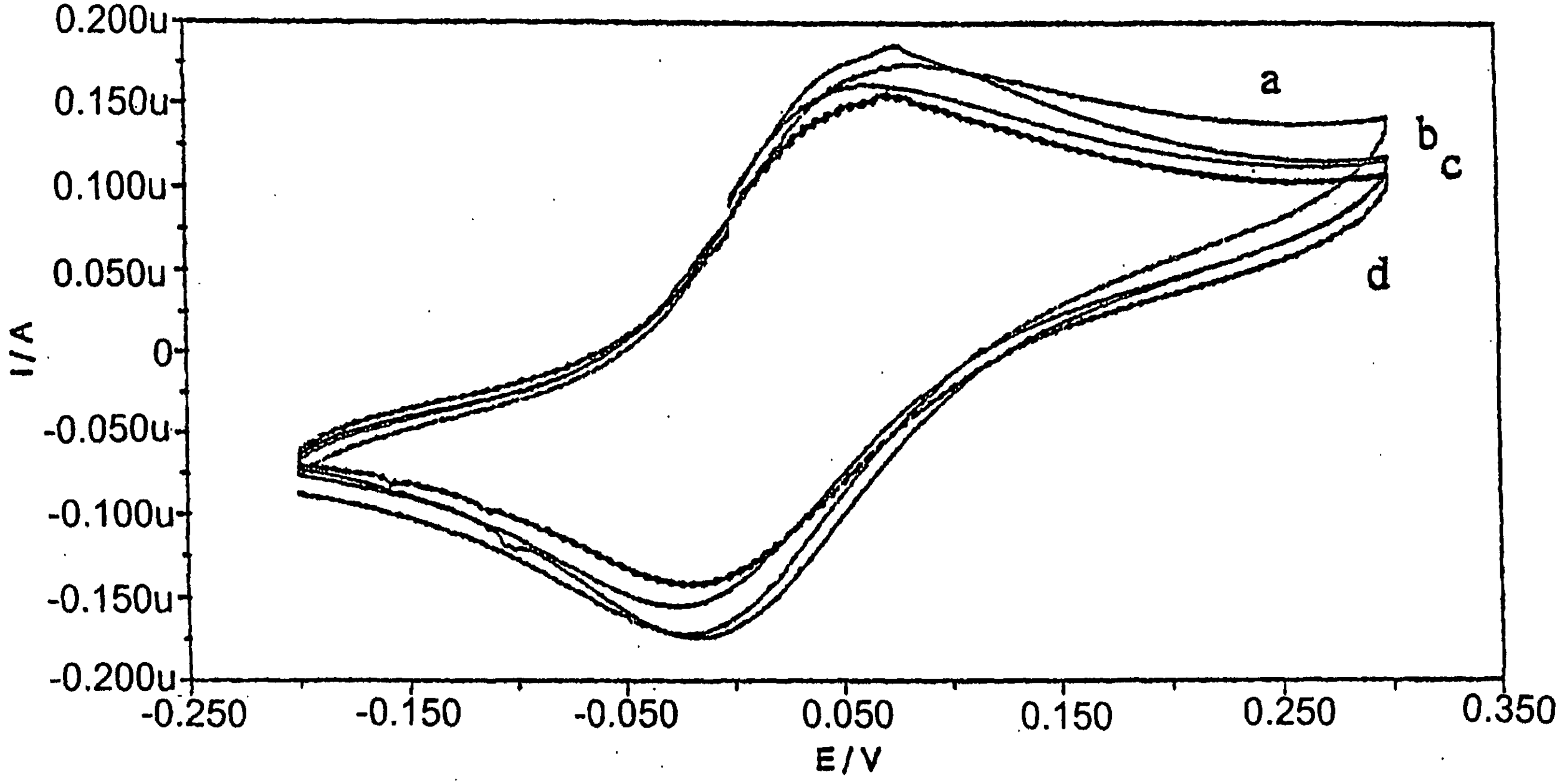


Abbildung 15: CV der TMPD-modifizierten Glukosebiosensoren mit Stärke-Schutzschicht, gemessen an unterschiedlichen Tagen, in glukosefreiem PBS-Puffer, (pH 7.4 ; $c_{(Cl^-)}$ 154 mM), v = 50 mV/s

a: 1. Tag ; b: 9. Tag ; c: 18. Tag ; d: 46. Tag

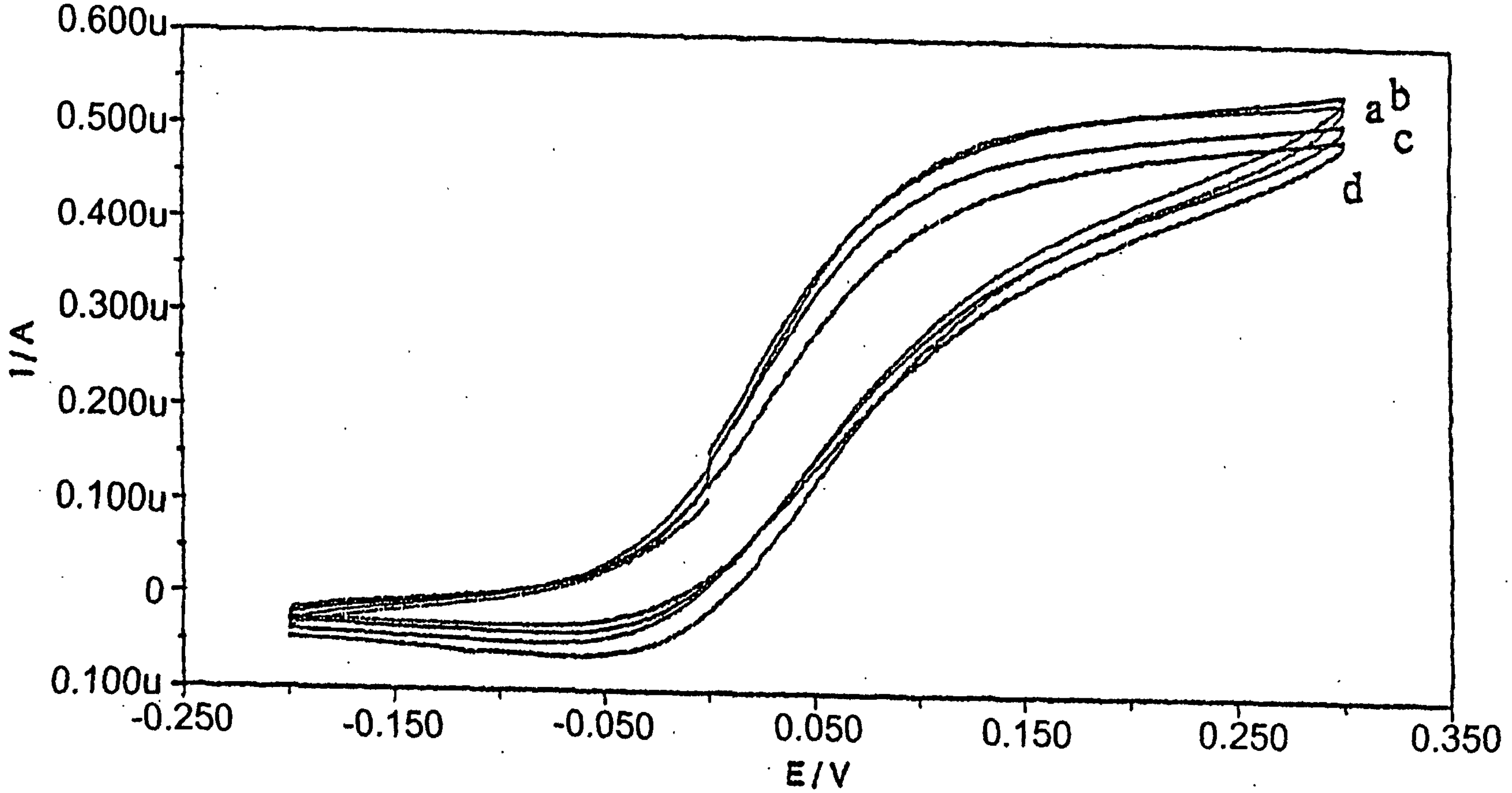


Abbildung 16:CV der TMPD-modifizierten Glukosebiosensoren mit Stärke-Schutzschicht, gemessen an unterschiedlichen Tagen, in gepufferter Glukoselösung, (pH 7.4 ; $c_{(Glukose)}$: 15 mM, $c_{(Cl^-)}$: 154 mM), v = 50 mV/s

a: 1. Tag ; b: 9. Tag ; c: 18. Tag ; d: 46. Tag

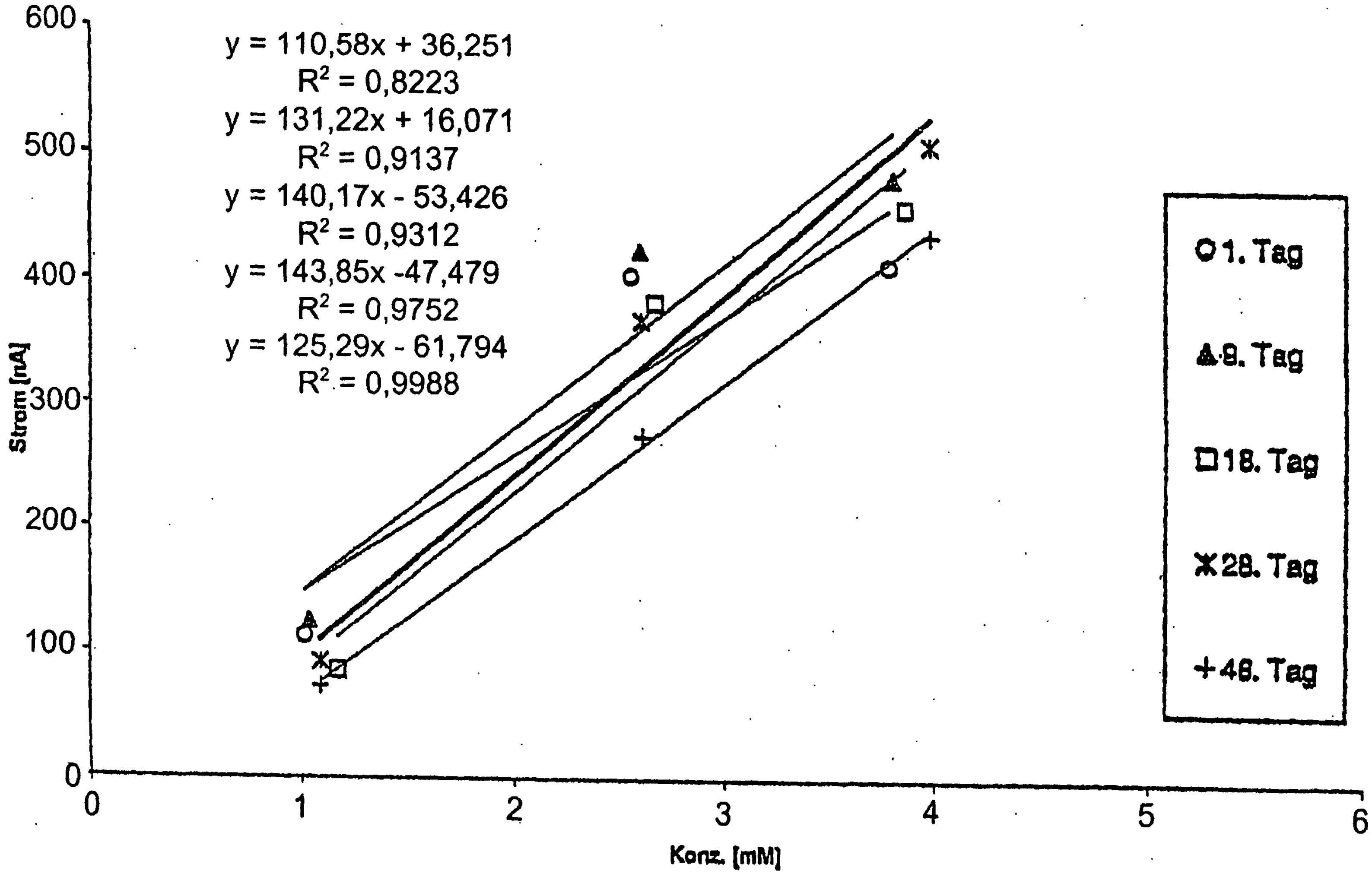


Abbildung 17: Sensitivität von TMPD-modifizierten Glukosebiosensoren mit Stärke-Schutzschicht in Abhängigkeit des Sensoralters

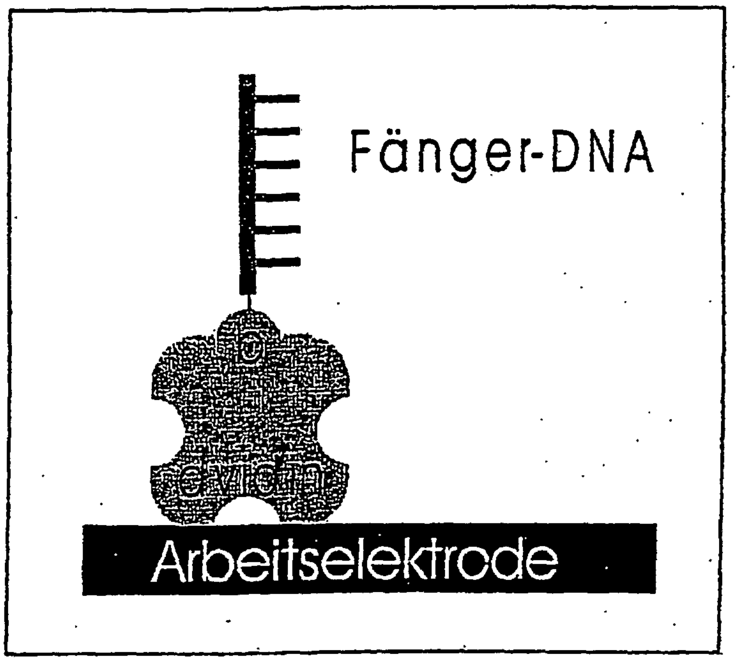


Abbildung 18: Immobilisierung der einzelsträngigen DNA als analyterkennendes Element über Avidin auf der Arbeitselektrode

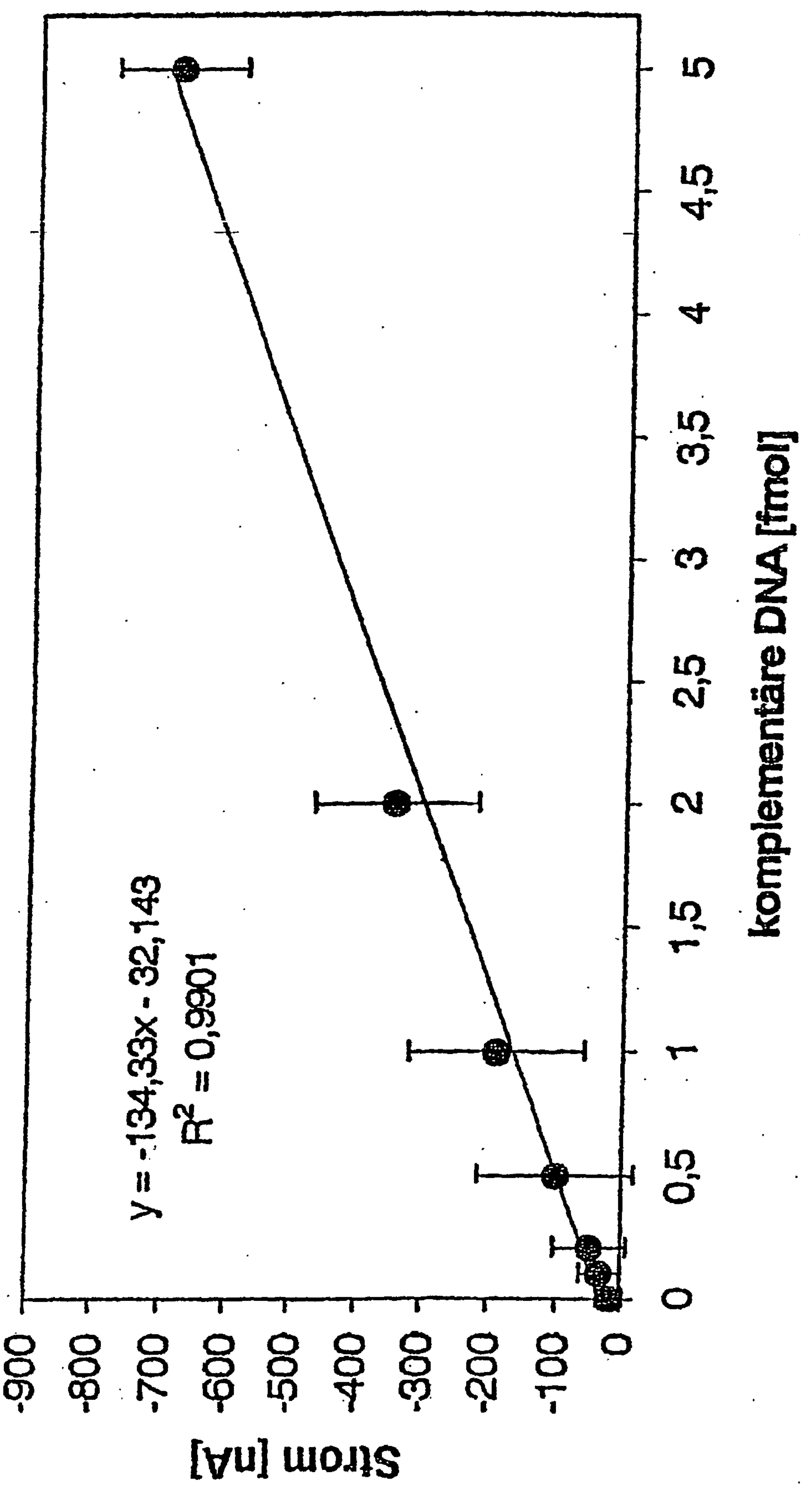


Abbildung 19: Kalibriergerade eines 20mer Oligonukleotids

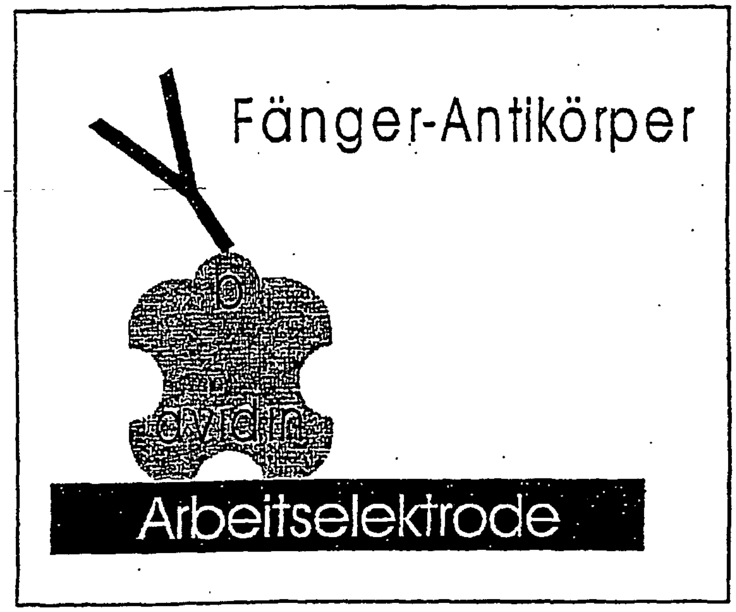


Abbildung 20: Immobilisierung des Fänger-Antikörpers als analyterkennendes Element über Avidin auf der Arbeitselektrode

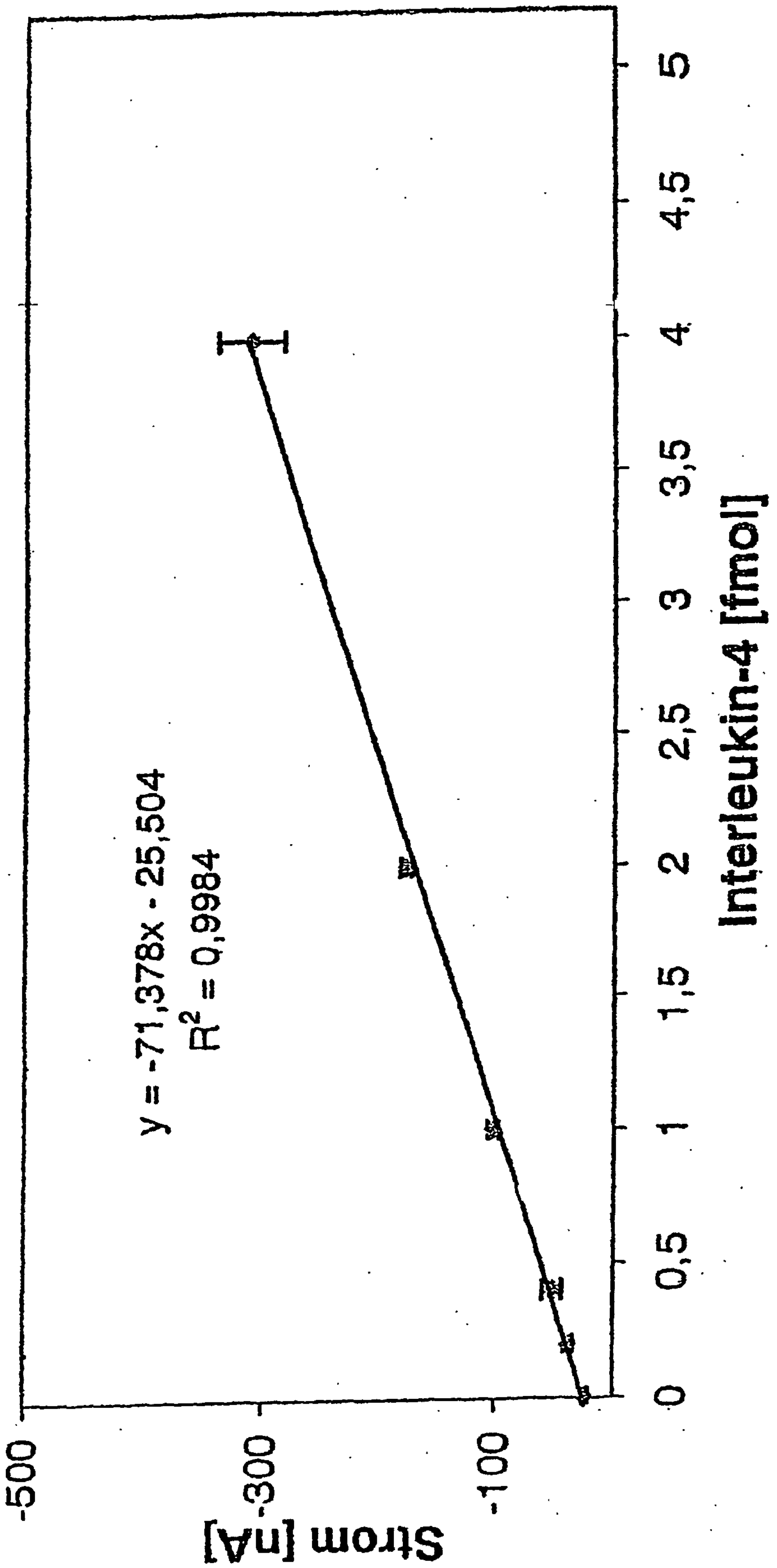


Abbildung 21: Kalibriergerade von Interleukin-4